# EUROPEAN PATENT SPECIFICATION

(11) **EP 4 618 849 B1**
(45) Date of publication and mention of the grant of the patent: **09.12.2026**
(21) Application number: 23836701.5
(22) Date of filing: 13.12.2023
(51) Int. Cl.: A61B 10/02

(54) **MULTI-SAMPLE CORE NEEDLE BIOPSY DEVICE HAVING LIMITED PIERCER FIRING**
BIOPSIEVORRICHTUNG FÜR MEHRPROBENKERNNADEL MIT BEGRENZTER DURCHSTECHZÜNDUNG
DISPOSITIF DE BIOPSIE À AIGUILLE À NOYAU À ÉCHANTILLONS MULTIPLES À DÉCLENCHEMENT DE PERÇAGE LIMITÉ

(30) Priority: 28.12.2022 US 202263435615 P
(43) Date of publication of application: 24.09.2025
(73) Proprietor: Devicor Medical Products, Inc., Cincinnati, OH 45241 (US)
(72) Inventor: MCBREEN, David, C., West Chester, OH 45069 (US); REBELLINO, Justin, Cincinnati, OH 45209 (US); NOCK, Andrew, P., Dayton, OH 45449 (US)
(74) Representative: Maiwald GmbH
(86) International application number: PCT/US2023/083826
(87) International publication number: WO 2024/145009

(56) References cited:
- US-A1- 2007 032 741
- US-A1- 2018 153 529
- US-A1- 2019 374 210
- US-A1- 2022 249 075

## Description

### BACKGROUND

A biopsy is the removal of a tissue sample from a patient to enable examination of the tissue for signs of cancer or other disorders. Tissue samples may be obtained in a variety of ways using various medical procedures involving a variety of the sample collection devices. For example, biopsies may be open procedures (surgically removing tissue after creating an incision) or percutaneous procedures (e.g. by fine needle aspiration, core needle biopsy, or vacuum assisted biopsy). After the tissue sample is collected, the tissue sample is typically analyzed at a lab (e.g. a pathology lab, biomedical lab, etc.) that is set up to perform the appropriate tests (such as histological analysis).

One technique for collecting a breast biopsy is to use a core needle biopsy device. One such device is the MAX-CORE disposable core biopsy instrument manufactured by Bard Biopsy Systems. Core needle biopsy devices frequently use a sharp, solid piercer equipped with a lateral tissue receiving notch positioned adjacent to the distal end of the piercer. When tissue is received within the notch, an elongate hollow cutting sheath is translated over the notch to sever a tissue sample. The severed tissue sample is then stored within the notch until both the piercer and the cutting sheath are removed from the patient. Thus, in core-needle biopsy devices, only one tissue sample can be collected per insertion of the piercer and cutting sheath.

In contrast to core needle breast biopsy procedures, vacuum-assisted breast biopsy devices permit a needle to remove multiple samples without requiring the needle be removed from the breast after every sample is collected. For instance, in a vacuum assisted breast biopsy device, a hollow needle is used to penetrate tissue. The hollow needle may include a lateral aperture adjacent to a sharp distal tip. A hollow cutter may be disposed within the hollow needle and may be moved axially relative to the lateral aperture of the needle to sever tissue samples. Once a tissue sample is severed by the hollow cutter, the tissue sample is transported axially though the cutter and collected in a tissue collection feature.

Examples of vacuum assisted biopsy devices and biopsy system components are disclosed in U.S. Pat. No. 5,526,822, entitled "Method and Apparatus for Automated Biopsy and Collection of Soft Tissue," issued June 18, 1996; U.S. Pat. No. 6,086,544, entitled "Control Apparatus for an Automated Surgical Biopsy Device," issued July 11, 2000; U.S. Pat. No. 6,162,187, entitled "Fluid Collection Apparatus for a Surgical Device," issued December 19, 2000; U.S. Pat. No. 6,432,065, entitled "Method for Using a Surgical Biopsy System with Remote Control for Selecting an Operational Mode," issued August 13, 2002; U.S. Pat. No. 6,752,768, entitled "Surgical Biopsy System with Remote Control for Selecting an Operational Mode," issued June 22, 2004; U.S. Pat. No. 7,442,171, entitled "Remote Thumbwheel for a Surgical Biopsy Device," issued October 8, 2008; U.S. Pat. No. 7,854,706, entitled "Clutch and Valving System for Tetherless Biopsy Device," issued December 1, 2010; U.S. Pat. No. 7,914,464, entitled "Surgical Biopsy System with Remote Control for Selecting an Operational Mode," issued March 29, 2011; U.S. Pat. No. 7,938,786, entitled "Vacuum Timing Algorithm for Biopsy Device," issued May 10, 2011; U.S. Pat. No. 8,083,687, entitled "Tissue Biopsy Device with Rotatably Linked Thumbwheel and Tissue Sample Holder," issued December 21, 2011; U.S. Pat. No. 8,118,755, entitled "Biopsy Sample Storage," issued February 1, 2012; U.S. Pat. No. 8,206,316, entitled "Tetherless Biopsy Device with Reusable Portion," issued on June 26, 2012; U.S. Pat. No. 8,702,623, entitled "Biopsy Device with Discrete Tissue Chambers," issued on April 22, 2014; U.S. Pat. No. 8,858,465, entitled "Biopsy Device with Motorized Needle Firing," issued October 14, 2014; and U.S. Patent No. 9,326,755, entitled "Biopsy Device Tissue Sample Holder with Bulk Chamber and Pathology Chamber," issued May 3, 2016.

Additional examples of vacuum assisted biopsy devices and biopsy system components are disclosed in U.S. Pub. No. 2006/0074345, entitled "Biopsy Apparatus and Method," published April 6, 2006 and now abandoned; U.S. Pub. No. 2009/0131821, entitled "Graphical User Interface for Biopsy System Control Module," published May 21, 2009, now abandoned; U.S. Pub. No. 2010/0152610, entitled "Hand Actuated Tetherless Biopsy Device with Pistol Grip," published June 17, 2010, now abandoned; U.S. Pub. No. 2010/0160819, entitled "Biopsy Device with Central Thumbwheel," published June 24, 2010, now abandoned; and U.S. Pub. No. 2013/0324882, entitled "Control for Biopsy Device," published December 5, 2013.

Examples of core needle biopsy devices are disclosed in U.S. Pat. No. 5,560,373, entitled "Needle Core Biopsy Instrument with Durable or Disposable Cannula Assembly," issued on October 1, 1996; U.S. Pat. No. 5,817,033, entitled "Needle Core Biopsy Device," issued on October 6, 1998; U.S. Pat. No. 5,971,939, entitled "Needle Core Biopsy Device," issued on October 26, 1999; and U.S. Pat. No. 5,511,556, entitled "Needle Core Biopsy Instrument," issued on April 30, 1996.

Examples of other forms of biopsy devices are disclosed in U.S. Pub. No. 2021/0153850, entitled "Tissue Collection Device for Collection of Tissue Samples from a Biopsy Needle and a Biopsy Device Including Tissue Collection Device," published on May 27, 2021; U.S. Pat. No. 8,485,989, entitled "Biopsy Apparatus Having a Tissue Sample Retrieval Mechanism," issued on July 16, 2013; and U.S. Pat. No. 11,013,499, entitled "Core Needle Biopsy Device," issued on May 25, 2021.

In some circumstances, it may be desirable to combine features from a core needle biopsy device and a vacuum assisted biopsy device to obtain the advantage of both devices and also reduce the overall disadvantages. For instance, core needle biopsy devices may be advantageous for their simplicity, light weight, and maneuverability. Furthermore, core needle biopsy devices generally include smaller sized needles, which can be desirable to increase patient comfort and recovery times. Meanwhile, vacuum assisted biopsy devices may be advantageous for their ability to collect multiple samples in a single insertion. Thus, a simple and light weight biopsy device capable of collecting multiple samples with a single insertion may be desirable.

One challenge in use of biopsy devices generally may include collecting one or more tissue samples from regions near particularly sensitive tissue. For instance, in the context of breast biopsy, collecting one or more tissue samples from the region near the axilla or the chest wall may present challenges. In such circumstances, it may be desirable to get close enough to sensitive anatomy to access regions of interest, yet avoid contact with sensitive anatomy with one or more portions of the biopsy device. This desirability can be complicated by the fact that elements of the biopsy device may be configured to fire such as the cutter and piercer in the circumstance of core needle biopsy. Thus, it may be desirable in some circumstances to limit or modify the firing elements of a biopsy device.

While several systems and methods have been made and used for obtaining a biopsy sample, it is believed that no one prior to the inventors has made or used the invention described in the appended claims.

US2022/0249075 discloses a core needle biopsy device using lead screws to move cutter and piercer drive assemblies.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements. In the drawings some components or portions of components are shown in phantom as depicted by broken lines.
FIG. 1 depicts a perspective view of a version of a core needle biopsy device.
FIG. 2 depicts a perspective view of a needle assembly of the core needle biopsy device of FIG. 1.
FIG. 3 depicts a side elevational view of the needle assembly of FIG. 2 with the needle assembly proximate a lesion.
FIG. 4 depicts another side elevational view of the needle assembly of FIG. 2 with a piercer of the needle assembly penetrating the lesion of FIG. 3.
FIG. 5 depicts yet another side elevational view of the needle assembly of FIG. 2 with a cutter of the needle assembly penetrating the lesion of FIG. 3 to sever a tissue sample.
FIG. 6 depicts a perspective view of the biopsy device of FIG. 1 with the piercer of FIG 4 being retracted relative to the cutter of FIG. 5.
FIG. 7 depicts a perspective view of a drive assembly that may be readily incorporated into the core needle biopsy device of FIG. 1.
FIG. 8A depicts a perspective view of a piercer drive assembly of the drive assembly of FIG. 7.
FIG. 8B depicts a detailed perspective view of a piercer lead screw and cutter lead screw of the piercer drive assembly of FIG. 8A.
FIG. 9 depicts an exploded perspective view of a latch mechanism of the piercer drive assembly of FIG. 8A.
FIG. 10 depicts a front elevational view of a cam member of the latch mechanism of FIG. 9.
FIG. 11 depicts a rear elevational view of the cam member of FIG. 10.
FIG. 12 depicts an exploded perspective view of a cutter drive assembly of the drive assembly of FIG. 7.
FIG. 13 depicts a perspective view of a control shaft of the drive assembly of FIG. 7.
FIG. 14 depicts a perspective cutaway view of the drive assembly of FIG. 7 incorporated into the core needle biopsy device of FIG. 1, the drive assembly in an initial configuration.
FIG. 15 depicts a perspective view of the latch mechanism of FIG. 9 coupled to the cutter drive assembly of FIG. 12.
FIG. 16 depicts another perspective cutaway view of the drive assembly of FIG. 7 incorporated into the core needle biopsy device of FIG. 1, the drive assembly in an armed configuration.
FIG. 17A depicts a cross-sectional view of the latch mechanism of FIG. 9 with the cross-section taken along line 17-17 of FIG. 16, the latch mechanism in a latched configuration.
FIG. 17B depicts another cross-sectional view of the latch mechanism of FIG. 9 with the cross-section taken along line 17-17 of FIG. 16, the latch mechanism in an unlatched configuration.
FIG. 18A depicts a cross-sectional view of the latch mechanism of FIG. 9 with the cross-section taken along a line perpendicular to line 17-17 of FIG. 16, the latch mechanism in the latched configuration.
FIG. 18B depicts another cross-sectional view of the latch mechanism of FIG. 9 with the cross-section taken along a line perpendicular to line 17-17 of FIG. 16, the latch mechanism in an intermediate configuration.
FIG. 18C depicts yet another cross-sectional view of the latch mechanism of FIG. 9 with the cross-section taken along a line perpendicular to line 17-17 of FIG. 16, the latch mechanism in the unlatched configuration.
FIG. 19 depicts yet another perspective cutaway view of the drive assembly of FIG. 7 incorporated into the core needle biopsy device of FIG. 1, the drive assembly in a fired configuration.
FIG. 20 depicts a perspective view of another drive assembly that may be readily incorporated into the core needle biopsy device of FIG. 1.
FIG. 21 depicts an exploded perspective view of a piercer drive assembly of the drive assembly of FIG. 20.
FIG. 22 depicts a perspective cross-sectional view of a lead screw shaft of the piercer drive assembly of FIG. 21, the cross-sectional taken along the longitudinal axis of the lead screw shaft.
FIG. 23 depicts a perspective view of a piercer carriage of the piercer drive assembly of FIG. 21.
FIG. 24 depicts an exploded perspective view of a latch mechanism of the piercer drive assembly of FIG. 21.
FIG. 25 depicts a front elevational view of a cam member of the latch mechanism of FIG. 24.
FIG. 26 depicts a rear elevational view of the cam member of FIG. 25.
FIG. 27 depicts an exploded perspective view of a cutter drive assembly of the drive assembly of FIG. 20.

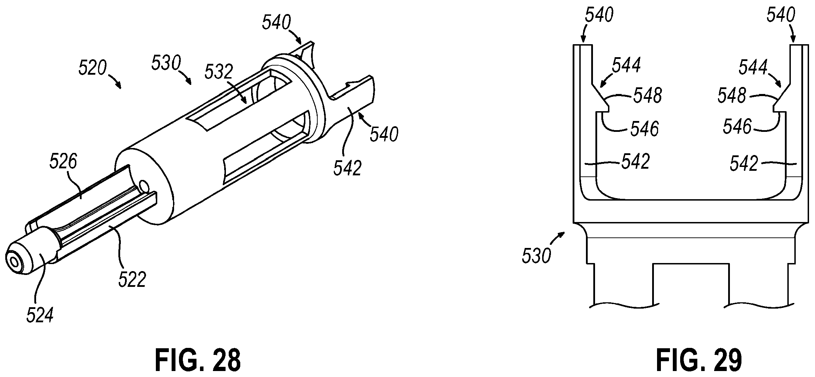

FIG. 28 depicts a perspective view of a cutter carriage of the cutter drive assembly of FIG. 27.
FIG. 29 depicts a partial top plan view of the cutter carriage of FIG. 28.
FIG. 30 depicts a perspective view of a control shaft of the drive assembly of FIG. 20.
FIG. 31 depicts a perspective cutaway view of the drive assembly of FIG. 20 incorporated into the core needle biopsy device of FIG. 1, the drive assembly in an initial configuration.
FIG. 32 depicts a perspective view of the latch mechanism of FIG. 24 coupled to the cutter drive assembly of FIG. 27.
FIG. 33 depicts a perspective view of a piercer of the core needle biopsy device of FIG. 1 positioned within a portion of the cutter carriage of FIG. 28.
FIG. 34 depicts another perspective cutaway view of the drive assembly of FIG. 20 incorporated into the core needle biopsy device of FIG. 1, the drive assembly in an armed configuration.
FIG. 35 depicts a side cross-sectional view of the drive assembly of FIG. 20 with the cross-section taken along line 35-35 of FIG. 34, the drive assembly in a short stop configuration.
FIG. 36 depicts a perspective view of the needle assembly of FIG. 2, the needle assembly in a short stop configuration corresponding to the short stop configuration of FIG. 35.
FIG. 37 depicts another side cross-sectional view of the drive assembly of FIG. 20 with the cross-section taken along the longitudinal axis of the drive assembly, the drive assembly in the armed configuration of FIG. 34.
FIG. 38 depicts another perspective view of the needle assembly of FIG. 2, the needle assembly in an armed configuration corresponding to the armed configuration of FIG. 34.
FIG. 39A depicts a cross-sectional view of the latch mechanism of FIG. 24 with the cross-section taken along line 39-39 of FIG. 34, the latch mechanism in a latched configuration.
FIG. 39B depicts another cross-sectional view of the latch mechanism of FIG. 24 with the cross-section taken along line 39-39 of FIG. 34, the latch mechanism in an intermediate configuration.
FIG. 40A depicts a cross-sectional view of the latch mechanism of FIG. 24 with the cross-section taken along a line perpendicular to line 39-39 of FIG. 34, the latch mechanism in the latched configuration.
FIG. 40B depicts another cross-sectional view of the latch mechanism of FIG. 24 with the cross-section taken along a line perpendicular to line 39-39 of FIG. 34, the latch mechanism in an unlatched configuration.
FIG. 41 depicts yet another perspective cutaway view of the drive assembly of FIG. 20 incorporated into the core needle biopsy device of FIG. 1, the drive assembly in a fired configuration.
FIG. 42 depicts a cross-sectional view of the cutter carriage of FIG. 28 with the cross-section taken along a line perpendicular to the longitudinal axis of the cutter carriage, the cutter carriage transitioning from an armed configuration to a fired configuration.
FIG. 43 depicts still another perspective view of the needle assembly of FIG. 2, the needle assembly in a fired configuration corresponding to the fired configuration of FIG. 41.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

Biopsy devices may be used to collect tissue samples in a variety of ways. For example, in some instances tissue samples are collected into a single tissue basket such that all tissue samples collected during a given biopsy procedure are deposited into the single tissue sample basket. In some other instances, tissue samples are collected into a tissue sample holder having separate compartments for each collected tissue sample. Such a multicompartment tissue sample holder may additionally include trays or strips that individually hold each tissue sample separately from the other tissue samples. Such trays or strips may be removable or otherwise separable from the tissue sample holder at the conclusion of a biopsy procedure.

Regardless of the structure in which the tissue samples are stored, tissue samples may be collected using biopsy devices under the guidance of various imaging modalities such as ultrasound image guidance, stereotactic (X-ray) guidance, MRI guidance, Positron Emission Mammography ("PEM" guidance), Breast-Specific Gamma Imaging ("BSGI") guidance, or otherwise. Each procedure has its own methodology based on the form of imaging guidance used.

Vacuum assisted biopsy devices and core needle biopsy devices both may have various advantages over the other, depending on context. For instance, one advantage of vacuum assisted biopsy devices is that vacuum assistance permits removal of multiple tissue samples using a single insertion. However, while core needle biopsy devices lack this feature, use of core needle biopsy devices may still be desirable in some circumstances. For instance, core needle biopsy devices may be generally capable of having smaller needles relative to vacuum assisted biopsy devices, thereby reducing patient anxiety and increasing the capacity of the needle to penetrate a lesion. Therefore, in some instances it may be desirable to incorporate the feature of multiple sample removal of a vacuum assisted biopsy device into a core needle biopsy device to achieve benefits present in both styles of biopsy device.

A desirable feature of the device described herein, which is a core needle biopsy device, is that the device allows for single insertion with multiple samples being obtained while using elements of a core needle biopsy device. To facilitate this functionality, the biopsy device further includes a drive mechanism to fire elements associated with a needle assembly such as a piercer and/or cutter. In some aspects, such drive mechanisms may be configured to limit the firing capacity of one or more elements of the needle to promote performance of biopsy procedures proximate sensitive patient anatomy.

### I. Example Core Needle Biopsy Device with Multi-Sample Collection

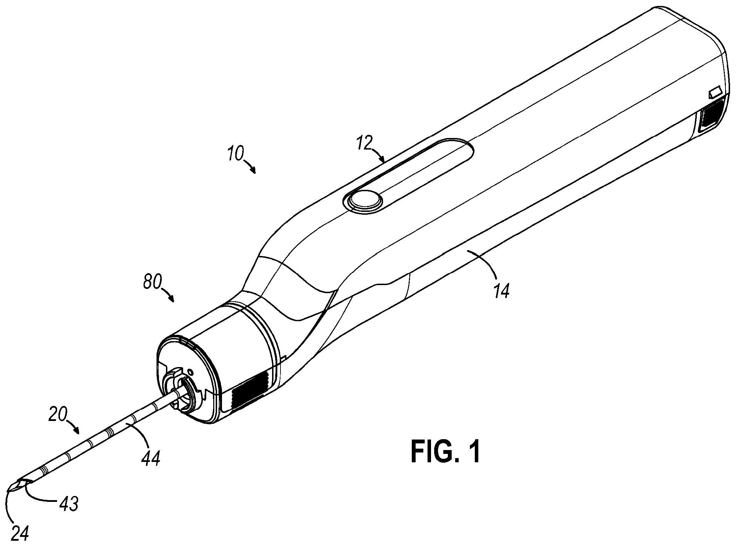

FIGS. 1 shows a version of a core needle biopsy device (10) for use in a breast biopsy procedure. Core needle biopsy device (10) of the present version comprises a body (12) and a needle assembly (20) extending distally from body (12). Body (12) includes an outer housing (14) and an actuation member (16) disposed on outer housing (14). As will be describe in greater detail below, outer housing (14) encloses various components of biopsy device (10), which are used to drive needle assembly (20) through a cutting cycle and a tissue acquisition cycle. To this end, outer housing (14) of the present version is sized and shaped for grasping by an operator using a single hand. Although not shown, it should be understood that in some versions outer housing (14) may comprise multiple parts such that each part interconnects to form outer housing (14).

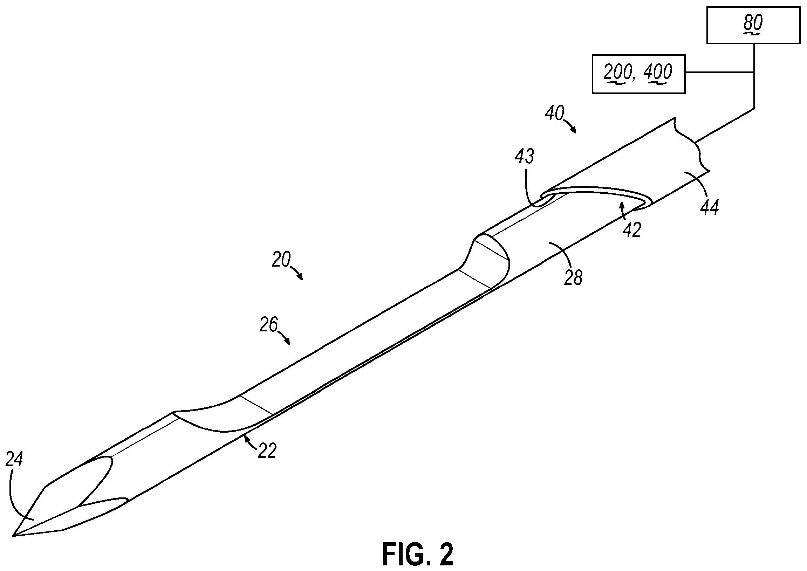

FIG. 2 shows needle assembly (20) in greater detail. As can be seen, needle assembly (20) comprises an elongate piercer (22) and an elongate cutter (40). As will be described in greater detail below, piercer (22) is generally movable relative to cutter (40) to pierce tissue and collect tissue samples, while cutter (40) is generally movable relative to piercer (22) to sever tissue samples. Piercer (22) comprises a generally cylindrical rod (28) (also referred to as a shaft) having a sharp distal tip (24) and a notch (26) disposed adjacent to distal tip (24). As will be described in greater detail below, distal tip (24) is generally configured to penetrate tissue of a patient. As will also be described in greater detail below, notch (26) is generally configured to receive tissue therein such that a tissue sample may be collected within notch (26) after the tissue sample is severed by cutter (40).

Cutter (40) comprises a generally hollow cylindrical tube that is configured to receive piercer (22) therein. Cutter (40) comprises an open distal end (42) and a cannula portion (44). Open distal end (42) is configured to permit at least a portion of piercer (22) to protrude from cutter (40) when piercer (22) is moved relative to cutter (40). In some versions, such as the version shown, open distal end (42) may also be oriented at an angle relative to the longitudinal axis of cutter (40). In other versions, open distal end (42) may alternatively be perpendicular relative to the longitudinal axis of cutter (40). As will be described in greater detail below, this configuration permits needle assembly (20) to move through the cutting cycle and the tissue acquisition cycle by permitting notch (26) of piercer (22) to move relative to distal end (42) of cutter (40).

Open distal end (42) of the present version includes a tapered edge (43). Tapered edge (43) is generally configured to slice through tissue to separate tissue samples when cutter (40) is moved relative to notch (26) of piercer (22). Thus, it should be understood that tapered edge (43) is generally configured to act a blade. Although the present version is described and shown as using a tapered configuration, it should be understood that in other versions various alternative configurations can be used. For instance, in some versions tapered edge (43) includes a plurality of serrations in addition or in alternative to the taper shown. In still other versions, tapered edge (43) can include any other additional or alternative cutting surface as will be apparent to those of ordinary skill in the art in view of the teachings herein.

Cannula portion (44) of cutter (40) extends proximally from distal end (42) and into an interior of body (12) such that piercer (22) can be received with the proximal end of cutter (40). In some versions, cannula portion (44) may be secured to an end portion, a cutter carriage or other feature used to promote manipulation of cutter (40). In such versions, features such as the end portion may be generally elongate to accommodate additional features such as tissue acquisition features. Suitable tissue acquisitions features may be configured in accordance with one or more of the teachings of U.S. Ser. No. 63/316,184, entitled "Sample Management for Core Needle Biopsy Device," filed on March 3, 2022

FIG. 2 shows piercer (22) disposed within cutter (40). As can be seen, cutter (40) is generally configured to receive piercer (22) such that piercer (22) is coaxial with cutter (40). In addition, piercer (22) is generally movable relative to open distal end (42) of cutter (40). It should be understood that in some circumstances, piercer (22) moves relative to cutter (40), while cutter (40) remains stationary. In other circumstances, cutter (40) moves relative to piercer (22), while piercer (22) remains stationary. In either case, it should be understood that piercer (22) and cutter (40) are generally configured such that notch (26) of piercer (22) moves into and out of cutter (40) such that notch (26) can be disposed distally or proximally relative to open distal end (42) of cutter (40). As will be described in greater detail below, this configuration permits piercer (22) and cutter (40) to operate cooperatively to pierce tissue, cut a tissue sample, and retract the tissue sample for collection by an operator via one or more tissue collection features.

As can also be seen in FIG. 2, cutter (40) and piercer (22) may be in communication with a tissue acquisition assembly (80) and a drive assembly (200, 400). As will be described in greater detail below, tissue acquisition assembly (80) and a given drive assembly (200, 400) may be configured to operate cooperatively with cutter (40) and piercer (22) to collect a plurality of tissue samples within a portion of tissue acquisition assembly (80) in a single insertion. For instance, a given drive assembly (200, 400) may be configured to move cutter (40) and piercer (22) in a predetermined sequence to sever a tissue sample. The given drive assembly (200, 400) may then retract piercer (22) relative to body (12) for manipulation of the severed tissue sample by sample acquisition assembly (80) out of notch (26) of piercer (22) and into a region of sample acquisition assembly (80) configured for tissue sample storage. In some versions, tissue acquisition assembly (80) may be configured in accordance with one or more of the teachings of U.S. Ser. No. 63/316,184, entitled "Sample Management for Core Needle Biopsy Device," filed on March 3, 2022. Although various aspects of drive assembly (200, 400) are described in greater detail below, it should be understood that in some versions, drive assembly (200, 400) may be configured in accordance with one or more of the teachings of U.S. Pub. No. 2022/0249075, entitled "Core Needle Biopsy Device for Collecting Multiple Samples in a Single Insertion," published on August 11, 2022.

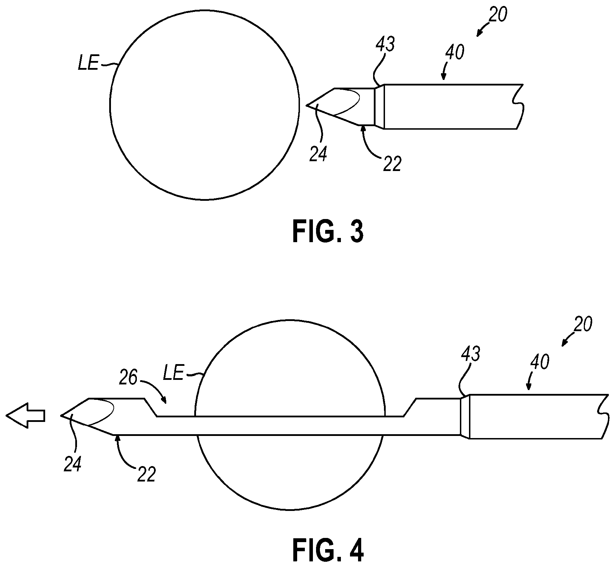

FIGS. 3 through 5 show an example process for collecting tissue samples using needle assembly (20). As can be seen in FIG. 3, needle assembly (20) may initially be positioned proximate a lesion (LE) or other region of interest. At this stage, cutter (40) may be positioned relative to piercer (22) so that cutter (40) covers notch (26) of piercer (22). Additionally, cutter (40) may further be positioned relative to piercer (22) such that sharp distal tip (24) of piercer (22) may protrude from open distal end (42) of cutter (40).

Once needle assembly (20) is positioned proximate relative to lesion (LE), piercer (22) may be advanced relative to cutter (40) as shown in FIG. 4. In some versions, piercer (22) may be rapidly fired into lesion (LE). In other versions, and as will be described in greater detail below, movement of piercer (22) may be less rapid. In still other versions, needle assembly (20) may be initially in the position shown in FIG. 4 (e.g., with piercer (22) advanced relative to cutter (40)) and needle assembly (20) may be inserted into lesion (LE) to the position shown in FIG. 4.

Regardless of the particular steps used to arrive at the position shown in FIG. 4, it should be understood that at this stage, needle assembly (20) is positioned to sever a tissue sample. In particular, with piercer (22) advanced relative to cutter (40), notch (26) is exposed relative to tissue. In this position, tissue may prolapse or otherwise enter or fill notch (26).

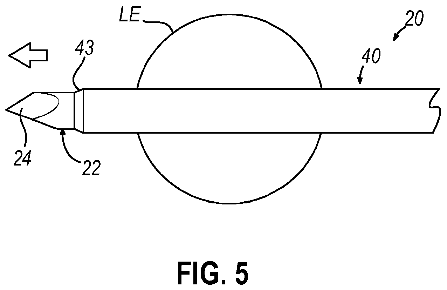

Once notch (26) is exposed relative to tissue, cutter (40) may be advanced relative to notch (26) to sever tissue using tapered edge (43). As best seen in FIG. 5, cutter (40) may be advanced relative to piercer (22) to completely cover notch (22). As cutter (40) is advanced, a tissue sample may be severed into notch (26). In some versions, cutter (40) may be advanced by any one of drive assemblies (200, 400) described herein at a relatively rapid rate. As will be described in greater detail below, such a relatively rapid rate may in some uses be referred to as firing under the action of a spring.

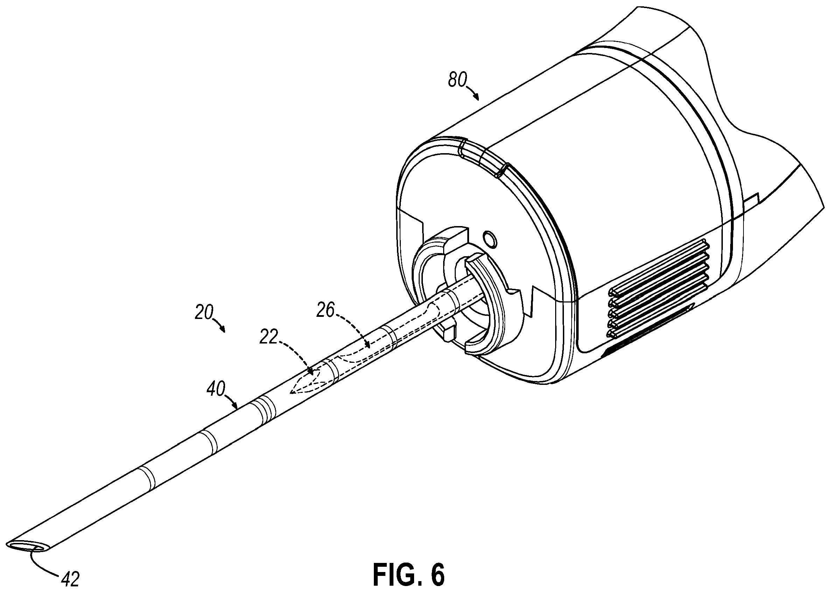

After advancement of cutter (40) to sever a tissue sample, piercer (22) may be retracted relative to cutter (40) as shown in FIG. 6. As can be seen, piercer (22) may be retraced proximally into tissue acquisition assembly (80), while cutter (40) remains generally stationary or otherwise in a distal position. Although not shown, it should be understood that one or more components of tissue acquisition assembly (80) may then remove the severed tissue sample from notch (26) for storage within one or more portions of tissue acquisition assembly. Additional tissue samples may then be collected by repeating the process described above with respect to FIGS. 3 through 6.

### II. Example Drive Assemblies for Core Needle Biopsy Device

As noted above, it may be desirable to incorporate a drive assembly into one or more portions of biopsy device (10) to facilitate manipulation of needle assembly (20) and/or portions of tissue acquisition assembly (80). Although suitable drive assemblies may take on a variety of forms, in some versions, it may be desirable for such drive assemblies to be configured to restrict certain movements of one or more portions of needle assembly (20). For instance, when biopsy device (10) is used to extract tissue samples from regions proximate sensitive anatomy, one or more portions of needle assembly (20) may have the propensity to contact the sensitive anatomy. To reduce or eliminate the likelihood of this contact, it may therefore be desirable to incorporate one or more features into suitable drive assemblies to limit or restrict certain movements of needle assembly (20).

### A. Example Drive Assembly with Axially Offset Drives

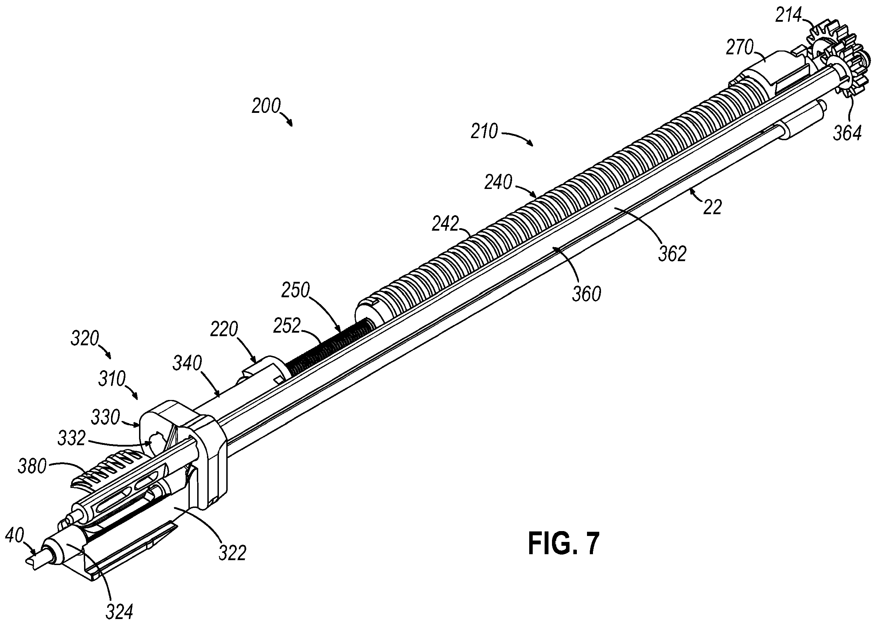

FIG. 7 shows a drive assembly (200) that may be readily incorporated into biopsy device (10) described above. As described above, drive assembly (200) may be generally configured to manipulate portions of needle assembly (20) such as piercer (22) and cutter (40). In some versions, this manipulation may include driving piercer (22) and/or cutter (40) through a predetermined sequence for cocking, firing, and sample collection.

To facilitate such sequential operation, drive assembly (200) includes a piercer drive assembly (210) and a cutter drive assembly (310). As will be described in greater detail below, piercer drive assembly (210) and cutter drive assembly (310) are generally interconnected with each other and generally configured to interact with each other. As a result of this interconnected configuration, pierce drive assembly (210) and cutter drive assembly (310) may operate to move piercer (22) and cutter (40) independently, while also in a predetermined sequence in a relatively compact size across the axial dimension.

Piecer drive assembly (210) may be in communication with piercer (22) such that piercer drive assembly (210) may be configured to drive piercer (22) through a predetermined sequence of movement independently from cutter (40), in concert with cutter (40), or both. Additionally, as will be described in greater detail below, piercer drive assembly (210) may be in communication with one or more elements of cutter drive assembly (310) to drive elements of cutter drive assembly (310) and/or cutter (40).

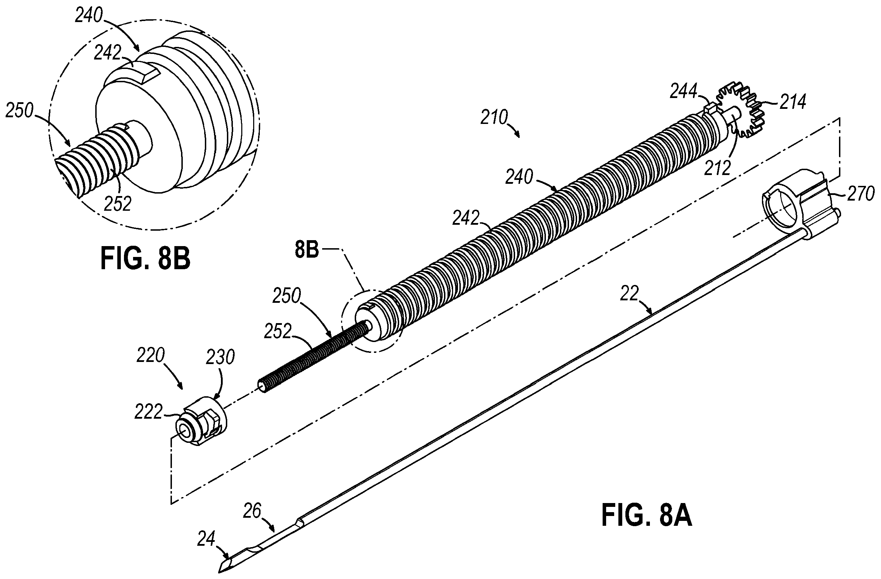

As best seen in FIG. 8A, piercer drive assembly (210) includes a lead screw drive shaft (212), a latch mechanism (220), a piercer lead screw (240) (also referred to as primary lead screw, or a piercer driver), a cutter lead screw (250) (also referred to as a secondary lead screw, or a cutter driver), and a piercer carriage (270). Generally, piercer drive assembly (210) is configured to move piercer (22) via piercer carriage (270) by moving piercer carriage (270) directly via rotation of piercer lead screw (240).

Lead screw drive shaft (212) is generally configured to drive rotation of piercer lead screw (240) and cutter lead screw (250). Lead screw drive shaft (212) extends proximally from piercer lead screw (240) and is generally fixedly secured to piercer lead screw (240) such that movement of lead screw drive shaft (212) corresponds to movement of piercer lead screw (240). Lead screw drive shaft (212) further includes a drive gear (214) that may be fastened thereto integral therewith to drive rotation of lead screw drive shaft (212). Thus, drive gear (214) may be configured to drive rotation of piercer lead screw (240) via lead screw drive shaft (212). Although not shown, it should be understood that drive gear (214) may mesh with other components of biopsy device (10) such as a motorized assembly to drive rotation of lead screw drive shaft (212).

Piercer lead screw (240) extends distally from lead screw drive shaft (212). Piercer lead screw (240) is generally cylindrical in shape and includes threading (242) and a hard stop (244) at a proximal end of threading (242). As will be understood, threading (242) may be generally configured to engage piercer carriage (270) to convert rotation of piercer lead screw (240) into translation of piercer (22) via piercer carriage (270). Similarly, hard stop (244) is configured to engage a portion of piercer carriage (270) to provide a mechanical stop to translation of piercer carriage (270) proximate the proximal end of piercer lead screw (240). Although not shown, it should be understood that in some versions, piercer lead screw (240) may additionally include a hard stop at a distal end of threading (242) similar to hard stop (244) described above.

Threading (242) in the present version is generally coarse such that rotation of piercer lead screw (240) may result in greater translation of piercer carriage (270) relative to other forms of threading described herein. Threading (242) of the present version is also of a left-handed configuration such that counterclockwise rotation (as viewed from the proximal end of piercer lead screw (240) looking distally) of piercer lead screw (240) may result in distal translation of piercer carriage (270). Although left-handed threading is used in the present version, it should be understood that in other versions this threading may be reversed. However, as will be described in greater detail below, such a reversal of threading (242) may result in corresponding reversal of other forms of threading associated with other components described herein.

Cutter lead screw (250) extends distally from piercer lead screw (240) along the longitudinal axis of piercer lead screw (240). Cutter lead screw (250) also defines a generally cylindrical shape similar to the cylindrical shape of piercer lead screw (240). However, the diameter of cutter lead screw (250) may generally be less than the diameter of piercer lead screw (240). It should be understood that cutter lead screw (250) may be fixedly secured or integral with piercer lead screw (240). Thus, rotation of piercer lead screw (240) may result in corresponding rotation of piercer lead screw (240).

Cutter lead screw (250) may also include threading (252). As will be understood, threading (252) of cutter lead screw (250) may be generally configured to engage latch mechanism (220) to convert rotation of cutter lead screw (250) into translation of latch mechanism (220) along the longitudinal axis of cutter lead screw (250).

Threading (252) of cutter lead screw (250) in the present version is generally fine relative to threading (242) of piercer lead screw (240). Thus, rotation of cutter lead screw (250) may generally result in less translation of latch mechanism (220) relative to translation of piercer carriage (270) generated by piercer lead screw (240).

Threading (252) of the present version is also opposite threading (242) of piercer lead screw (240). In other words, threading (252) of cutter lead screw (250) may be of a right-handed configuration such that counterclockwise rotation (as viewed from the proximal end of piercer lead screw (240) looking distally) of cutter lead screw (250) may result in proximal translation of latch mechanism (220). As will be understood, this opposing thread configuration may be desirable to promote certain sequential movements of piercer (22) and cutter (40). Although right-handed threading is used in the present version, it should be understood that in other versions, this threading may be reversed. However, as will be appreciated, such a reversal of threading (252) may result in corresponding reversal of threading (242) to still provide the opposing thread configuration described herein.

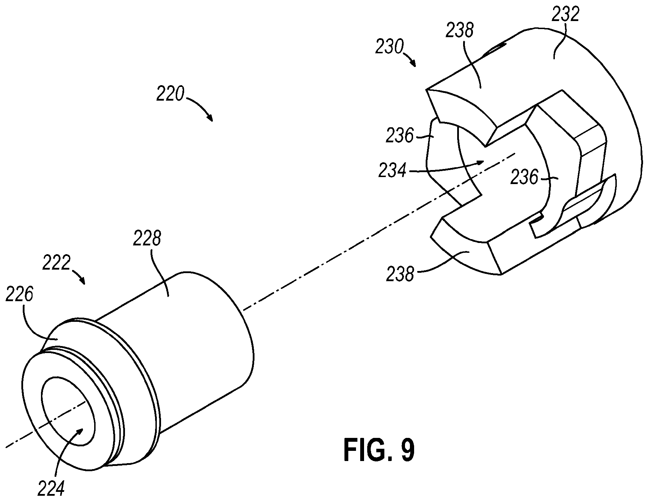

FIGS. 9 through 11 show latch mechanism (220) in greater detail. Although latch mechanism (220) is characterized herein as being a part of piercer drive assembly (210), latch mechanism (220) may also be characterized as being a part of cutter drive assembly (310), another portion of drive assembly (200), or a separate component entirely. As will be understood, latch mechanism (220) is generally configured to interact with various components of drive assembly (200) to facilitate interaction between piercer drive assembly (210) and cutter drive assembly (310).

Latch mechanism (220) includes an axial locator (222) (also referred to as a nut member) and a cam member (230). As will be described in greater detail below, axial locator (222) and cam member (230) are generally configured to work cooperatively to move along the longitudinal axis of cutter lead screw (250) and selectively engage and/or disengage one or more portions of cutter drive assembly (310).

As best seen in FIG. 9, axial locator (222) defines a generally cylindrical shape. Axial locator (222) defines a threaded bore (224), a collar portion (226), and a receiving portion (228). Threaded bore (224) may extend longitudinally through axial locator (222) and may include threading (not shown) configured to engage threading (252) of cutter lead screw (250). Thus, it should be understood that axial locator (222) may be configured to axially translate along the length of cutter lead screw (250) in response to rotation of cutter lead screw (250).

Both collar portion (226) and receiving portion (228) are configured to work cooperatively to receive and located cam member (230) relative to axial locator (222). Collar portion (226) extends outwardly from receiving portion (228) defining a structure similar to a flange. As will be described in greater detail below, collar portion (226) is generally configured to engage one or more portions of cutter drive assembly (310) to associate movement of latch mechanism (220) with cutter drive assembly (310). Optionally, a distal portion of collar portion (226) may be sloped, angled, or rounded to promote engagement with cutter drive assembly (310). Meanwhile, receiving portion (228) defines a generally smooth cylindrical surface configured for receipt within a portion of cam member (230), as will be described in greater detail below.

Cam member (230) is generally configured to receive axial locator (222) and to rotate relative to axial locator (222). Cam member (230) includes a body (232) defining a receiving bore (234), a one or more cam features (236) projecting from an outer surface of body (232), and one or more axial projections (238) extending axially from body (232). Receiving bore (234) is generally configured to receive at least a portion of axial locator (222), while cam features (236), and axial projections (238) are generally configured to promote manipulation of cam member (230) relative to axial locator (222) for engagement with one or more portions of cutter drive assembly (310).

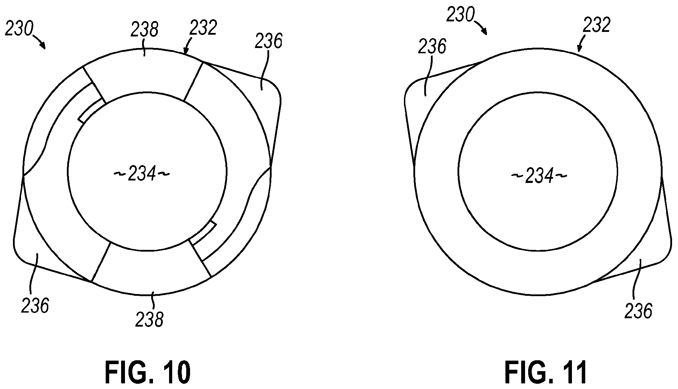

As best seen in FIGS. 10 and 11, cam member (230) of the present version includes a pair of cam features (236), although other suitable numbers may be used in other versions. Each cam feature (236) is generally configured to engage a portion of cutter drive assembly (310) upon rotation of cam member (230) as will be described in greater detail below. To facilitate such engagement, a portion of each cam member (230) may be sloped or contoured. On other words, each cam feature (236) may include a camming surface to drive movement of one or more portions of cutter drive assembly (310).

Cam member (230) of the present version likewise includes a pair of axial projections (238) corresponding to each cam feature (236). Each axial projection (238) may be disposed between each cam feature (236) about the circumference of cam member (230). As will be understood, this configuration may define a pocket or open space proximate each cam feature (236) to provide accessibility to both axial projections (238) and cam features (236). Specifically, such pockets or open space may permit extension of one or more portions of cutter drive assembly (310) into cam member (230) to engage each cam feature (236). Additionally, such pockets or open space may permit access to one or more surfaces of each axial projection (238) for manipulation of cam member (230) via one or more axial projections (238).

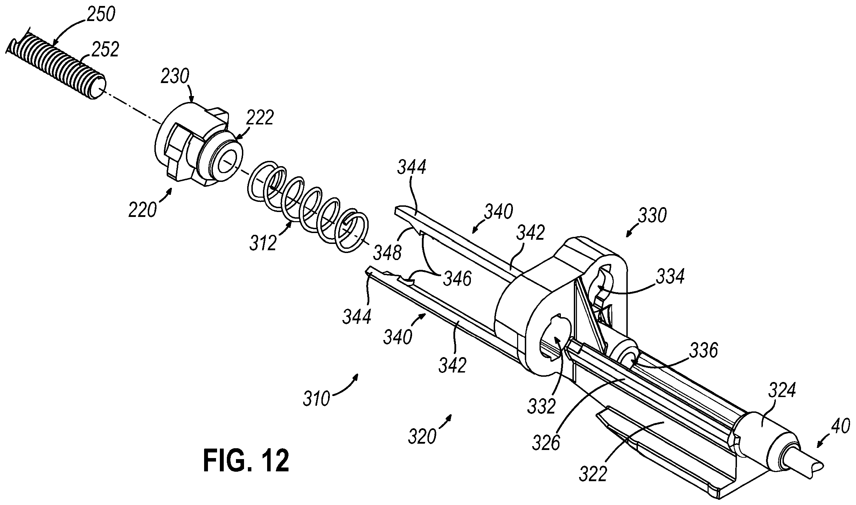

FIG. 12 shows cutter drive assembly (310) in greater detail. As can be seen, cutter drive assembly (310) includes a cutter spring (312) and a cutter carriage (320). Additionally, it should be understood that in some contexts, latch mechanism (220) may be characterized as a part of cutter drive assembly (310) rather than piercer drive assembly (210) because latch mechanism (220) may engage with elements of both assemblies (210, 310). As will be understood, cutter drive assembly (310) is generally configured to engage with piercer drive assembly (210) and other elements of drive assembly (200) to sequentially actuate and fire cutter (40).

Cutter carriage (320) includes a carriage body (322) that defines a cutter collar (324), tissue manipulator (326), and a proximal receiving end (330). Cutter collar (324) is configured to receive the proximal end of cutter (40) such that cutter (40) may extend distally from cutter collar (324). In the present version, cutter (40) may be fixedly secured to cutter collar (324). Alternatively, in other versions, cutter collar (324) may be threaded, keyed, or otherwise structured to permit cutter (40) to be removably secured to cutter collar (324). Cutter collar (324) is generally of a hollow configuration to promote access to the proximal end of cutter (40) by piercer (22) and/or other structures.

Tissue manipulator (326) is disposed between cutter collar (324) and proximal receiving end (330). Tissue manipulator (326) is generally configured to direct tissue from piercer (22) into a tissue sample chamber or other structure during operation of drive assembly (200). Thus, tissue manipulator (326) is configured to receive at least a portion of piercer (22) such that piercer (22) may extend and move through tissue manipulator (326). In some versions, tissue manipulator (326) may be used in connection with other features such as a flexible member, a wiper, or a blade feature. In such versions, tissue manipulator (326) and associated features may be configured in accordance with one or more teachings of U.S. Ser. No. 63/316,184, entitled "Sample Management for Core Needle Biopsy Device," filed on March 3, 2022.

Proximal receiving end (330) of carriage body (322) is generally configured to engage various components of drive assembly (200) to drive movement of cutter (40) via movement of cutter carriage (320). In particular, proximal receiving end (330) defines a driver bore (332), a shaft bore (334), and a piercer bore (336). Bores (332, 334, 336) may be configured to provide clearance for certain elements of drive assembly (200) and/or biopsy device (10) though cutter carriage (320), as will be described in greater detail below. Although bores (332, 334, 336) are shown in the present version as defining generally cylindrical passageways through a portion of cutter carriage (320), it should be understood that in other versions, any one or more of bores (332, 334, 336) may use various shapes or may be omitted entirely.

Proximal receiving end (330) further includes one or more retaining arms (340) extending proximally from a proximal surface of proximal receiving end (330). Retaining arms (340) are generally configured to resiliently engage a portion of latch mechanism (220) to permit selective coupling of cutter drive assembly (310) to piercer drive assembly (210), as will be described in greater detail below. Each retaining arm (340) includes a respective extension member (342) and engagement member (344). Each extension member (342) is generally configured to have some rigidity, but also some flexibility. As will be described in greater detail below, this combination of rigidity and flexibility may be desirable to promote selective coupling and decoupling with respect to latch mechanism (220).

Each engagement member (344) is generally configured as a tooth or catch. As will be understood, this configuration may permit each engagement member (344) to engage collar portion (226) of axial locator (222). Specifically, each engagement member (344) includes a flat face (346) and an angled surface (348) projecting proximally relative to flat face (346). As will be described in greater detail below, flat face (346) may be configured to engage collar portion (226) of axial locator (222) for fastening, while angled surface (348) may be configured to engage collar portion (226) for manipulation of each respective retaining arm (340) during fastening.

In the present configuration, proximal receiving end (330) includes a pair of retaining arms (340) oriented opposite each other around the perimeter of a circle corresponding to about the diameter of axial locator (222). In other versions, any suitable number of retaining arms (340) may be used such as three, four, or more. Alternatively, in still other versions, retaining arms (340) may be configured as a single element with relief slots formed in a structure thereof to promote similar functionality in a single element component.

Cutter spring (312) is configured to engage proximal receiving end (330) to drive cutter carriage (320) distally during firing of cutter (40). Specifically, cutter spring (312) includes a coil spring configured to be disposed between proximal receiving end (330), retaining arms (340) and latch mechanism (220). As will be described in greater detail below, cutter spring (312) is configured to be compressed between proximal receiving end (330) and latch mechanism (220) to store potential energy that may be released during firing of cutter (40).

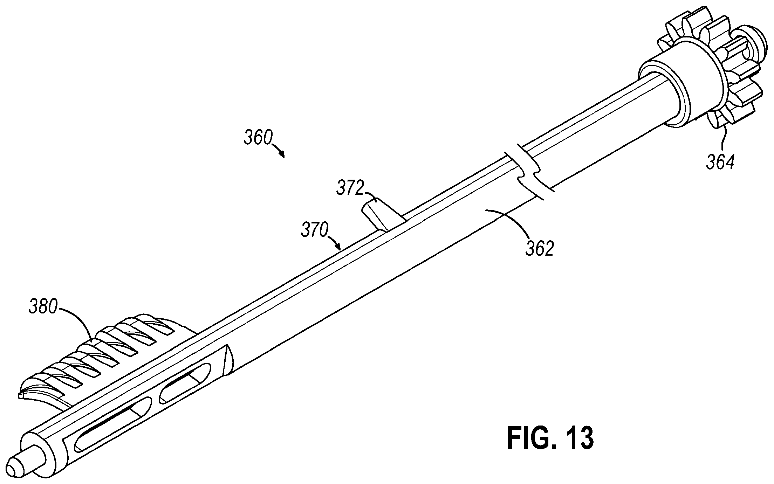

Drive assembly (200) further includes a control shaft (360) (also referred to as an actuation mechanism, control, or trip mechanism), which may be configured to interact with cutter drive assembly (310) to initiate movement of cutter (40) through a predetermined sequence of motion. As best seen in FIG. 13, control shaft (360) includes an elongate shaft (362) having a drive gear (364), a latch portion (370), and a tissue manipulation portion (380). Elongate shaft (362) defines a generally cylindrical cross-section and is configured to extend from piercer drive assembly (210) to cutter drive assembly (310).

Drive gear (364) is configured to engage a gear associated with a motor or other drive mechanism to drive rotation of control shaft (360). In some versions, drive gear (364) may be driven by the same motor used to drive gear (214) of piercer drive assembly (210) using a transmission, gearing, and/or other mechanisms. In other versions, drive gear (364) may be driven by a dedicated motor. In still other versions, drive gear (364) may be driven manually by a knob, thumbwheel, crank, or other manual drive mechanism.

Latch portion (370) is disposed along elongate shaft (362) between drive gear (364) tissue manipulation portion (380). Latch portion (370) is generally configured to engage a portion of latch mechanism (220) to control one or more operations of cutter drive assembly (310). In particular, latch portion (370) includes an actuator (372) protruding outwardly from elongate shaft (362). As will be described in greater detail below, actuator (372) is configured to engage one or more of axial projections (238) of cam member (230) to selectively release cutter carriage (320) from latch mechanism (220).

Tissue manipulation portion (380) is disposed proximate the distal end of elongate shaft (362). Tissue manipulation portion (380) is generally configured to manipulate a tissue sample out of piercer (22) and into a tissue chamber in response to rotation of control shaft (360). Tissue manipulation portion (380) may include a variety of features to facilitate such functionality. In some versions, tissue manipulation portion (380) may be configured in accordance with one or more teachings of U.S. Ser. No. 63/316,184, entitled "Sample Management for Core Needle Biopsy Device," filed on March 3, 2022.

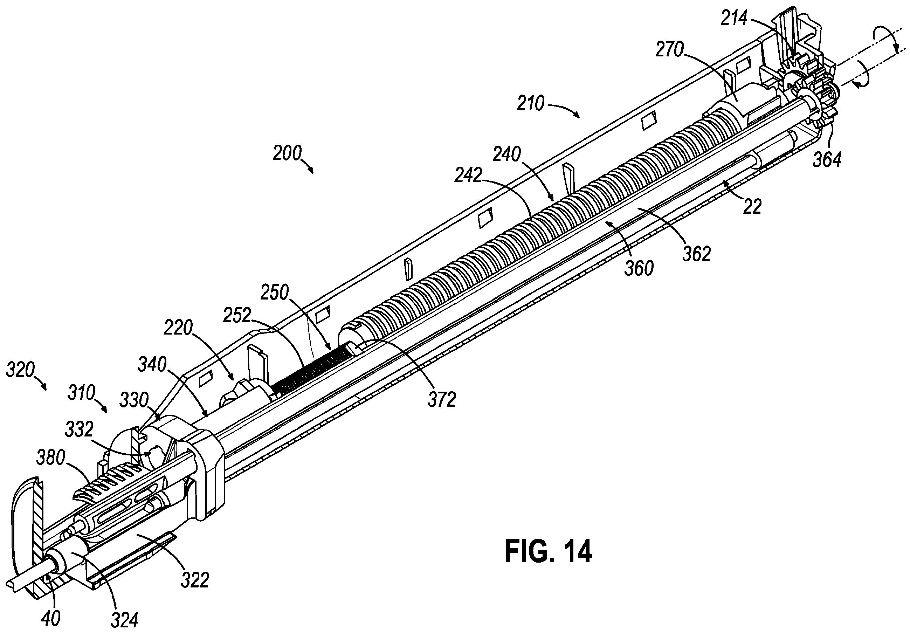

FIGS. 14 through 19 show an example use of drive assembly (200) in connection with biopsy device (10) to collect one or more tissue samples using a single insertion of needle assembly (20). As best seen in FIG. 14, drive assembly (200) may initially begin in an initial configuration (also referred to as a home or retrieve configuration). To transition to the initial configuration, piercer lead screw (240) may be rotated to drive piercer carriage (270) proximally via threading (242). The same rotation of piercer lead screw (240) may also drive distal translation of latch mechanism (220) along cutter lead screw (250) via threading (252), which may be opposite in pitch relative to threading (242).

Once rotation of piercer lead screw (240) is completed to position drive assembly (200) in the initial position, piercer carriage (270) may be fully retracted proximally relative to piercer lead screw (240). In this position, piercer (22) may be in a proximal-most position. In such a position, notch (26) may be aligned tissue manipulator (326) of cutter carriage (320). Thus, although the initial configuration may be used as a start or home position, the initial configuration may additionally be used as a sample collection position, as will be described in greater detail below.

Also in the initial position, cutter carriage (320) may be fully advanced distally relative to cutter lead screw (250). Specifically, latch mechanism (220) may advance distally via cutter lead screw (250), engage cutter carriage (320), and drive cutter carriage (320) distally to the initial position shown in FIG. 14. In this position, cutter (40) may be in a distal-most position (also referred to as a fired position). In some versions, cutter carriage (320) may abut a distal hard stop within the outer housing of biopsy device (10) to prevent further distal movement of cutter (40).

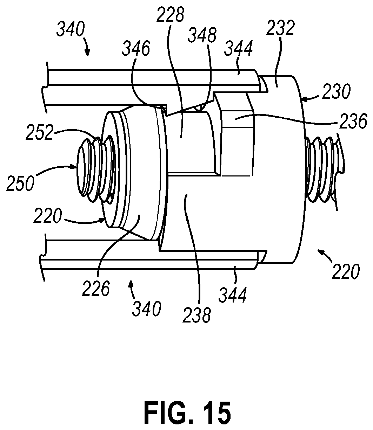

As described above, latch mechanism (220) may also be fully advanced distally via cutter lead screw (250). During distal advancement of latch mechanism (220), latch mechanism (220) may engage cutter carriage (320). As best seen in FIG. 15, latch mechanism (220) may contact retaining arms (340) of cutter carriage (320). As latch mechanism (220) is advanced further distally, collar portion (226) of axial locator (222) may engage angled surface (348) of each retaining arm (340), driving retaining arms (340) away from each other until collar portion (226) is disposed distally of flat face (346). Each flat face (346) may then engage collar portion (226) of axial locator (222), thereby coupling latch mechanism (220) to cutter carriage (320).

It should be understood that in some versions, coupling of latch mechanism (220) to cutter carriage (320) may also simultaneously compress cutter spring (312). In such uses, this may result in cutter (40) being characterized as partially armed. When partially armed, cutter spring (312) may be fully compressed in preparation for firing of cutter (40). However, due to the distal position of cutter carriage (320), actual firing of cutter (40) may be blocked or otherwise prevented.

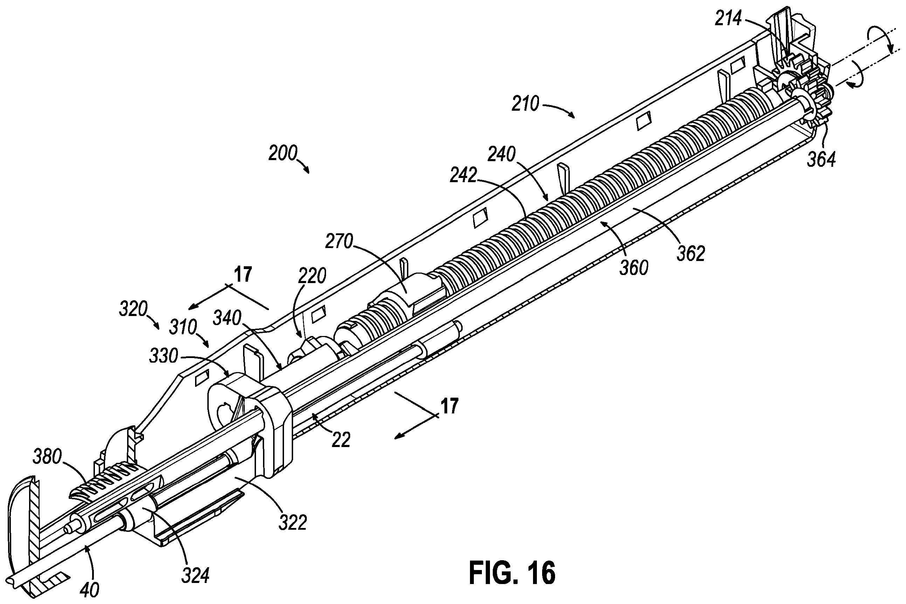

Once drive assembly (200) is in the initial configuration, it may be desirable to initiate arming of drive assembly (200). FIG. 16 shows drive assembly (200) in the armed configuration. In the present version, the transition from the initial configuration to the armed configuration may be initiated by an operator providing a user input (e.g., pressing a button on biopsy device (10)). Alternatively, in some versions, the transition to the armed configuration may be initiated automatically.

Regardless of the particular initiation process, the transition from the initial configuration to the armed configuration may begin by piercer lead screw (240) rotating in an opposite direction relative to the direction described above to translate piercer carriage (270) distally. Simultaneously, cutter lead screw (250) may rotate in the same direction as piercer lead screw (240) to translate latch mechanism (220) and cutter carriage (320) proximally. In other words, piercer carriage (270) and cutter carriage (320) may translate in opposite directions via the opposite hand threading of piercer lead screw (240) and cutter lead screw (250).

Rotation of piercer lead screw (240) and cutter lead screw (250) may continue until piercer carriage (270) reaches the distal position shown in FIG. 16 and cutter carriage (320) reaches the proximal position also shown in FIG. 16. With piercer carriage (270) in the distal position, piercer (22) may be in a distal-most position. Similarly, with cutter carriage (320) in the proximal position, cutter (40) may be in a proximal-most position. This may result in a relative position between piercer (22) and cutter (40) that includes notch (26) of piercer (22) being exposed relative to cutter (40).

In an alternative armed configuration, it may be desirable for notch (26) of piercer (22) to be covered by cutter (40). In such an alternative configuration, rotation of piercer lead screw (240) and cutter lead screw (250) may stop prior to piercer carriage (270) reaching the distal position. At this stage, further user input (e.g., a second press of a button) may be used to initiate further rotation of piercer lead screw (240) and cutter lead screw (250) to reach the respective distal and proximal positions shown in FIG. 16. In some versions, a series of light emitting diodes (LED) may be used to indicate a status of drive assembly (200) (e.g., one LED for armed, two LED for exposed notch (26), three LED for fired).

Retraction of cutter carriage (320) proximally via engagement with latch mechanism (220) may also serve to arm or cock cutter drive assembly (310) for firing. Specifically, in versions where cutter spring (312) is compressed during the transition to the initial configuration, arming or cocking may include retracting cutter carriage (320) proximally to provide space for subsequent distal advancement of cutter carriage (320) during firing of cutter (40).

In other versions, proximal translation of cutter carriage (320) may also be used for compression of cutter spring (312). Specifically, in some versions, cutter spring (312) may be in other positioned relative to cutter carriage (320) rather than being compressed between cutter carriage (320) and latch mechanism (220). By way of example only, in some versions, cutter spring (312) may be compressed between cutter carriage (320) and a portion of a housing of biopsy device (10). In such versions, compression of cutter spring (312) may be based on movement of cutter carriage (320) relative to the housing rather than the movement of cutter carriage (320) relative to latch mechanism (220). Thus, in such versions, proximal translation of cutter carriage (320) during arming or cocking may also include compression of cutter spring (312).

Once drive assembly (200) is moved into the armed configuration, piercer (22) and cutter (40) may be inserted into tissue and positioned proximate a lesion. Such insertion into tissue may be performed either with notch (26) of piercer (22) exposed relative to cutter (40) or notch (26) covered by cutter (40). Optionally, insertion at this stage may be performed in combination with various forms of image guidance such as ultrasonic, x-ray, magnetic resonance imaging (MRI), and/or etc.

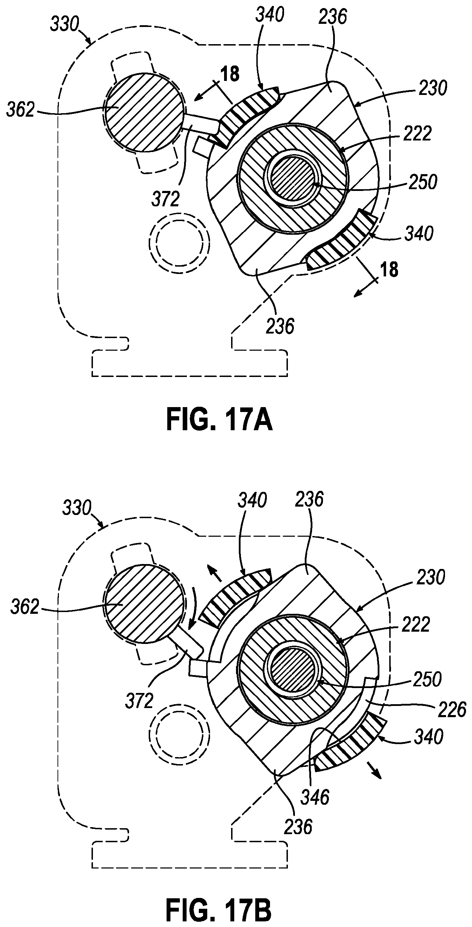

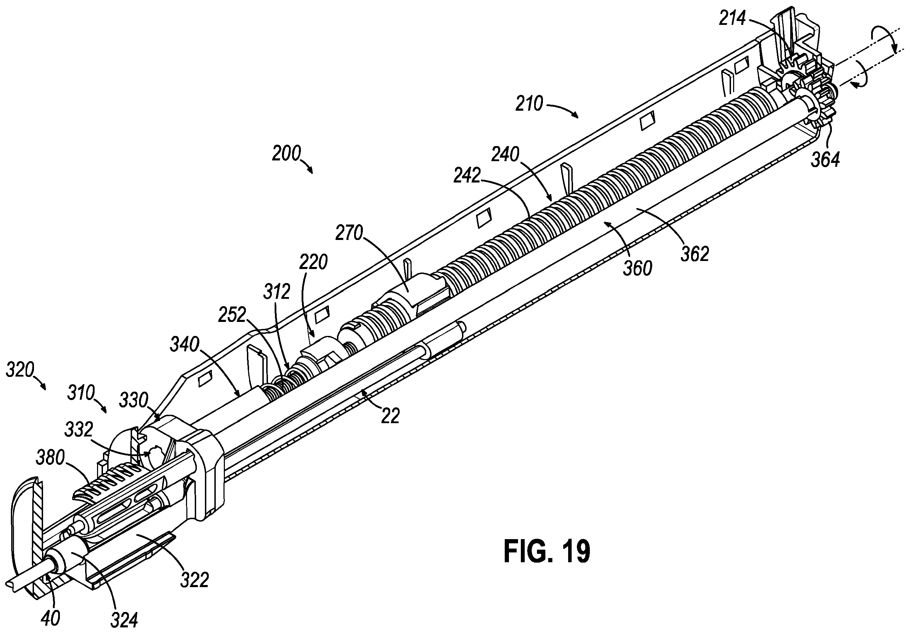

After drive assembly (200) is moved to the armed configuration, it may be desirable to fire cutter (40) relative to piercer (22) to sever a tissue sample into notch (26) of piercer (22). As similarly discussed above for the armed configuration, firing may be initiated using a user input such as a button. FIGS. 17A through 18C show a sequence that may occur to move drive assembly (200) into a fired configuration shown in FIG. 19.

As best seen in FIGS. 17A through 17B, the firing sequence may begin with control shaft (360) rotating to move actuator (372) of latch portion (370) toward latch mechanism (220). At this stage, actuator (372) may engage axial projections (238) or other suitable portions of cam member (230) to rotate cam member (230) relative to axial locator (222). As cam member (230) is rotated, cam features (236) may be driven into engagement with retaining arms (340) of cutter carriage (320).

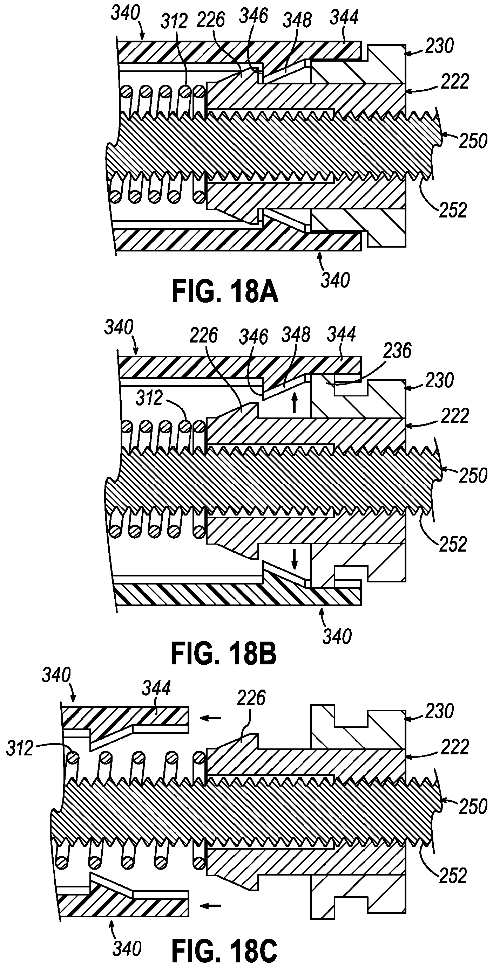

Further rotation of cam member (230) may cause retaining arms (340) of cutter carriage (320) to release from collar portion (226) of axial locator (222). Specifically, as best seen in FIGS. 18A through 18C, rotation of cam member (230) may cause cam features (236) to exert outward pressure on each retaining arm (340) of cutter carriage (320). As a result, flat face (346) of engagement member (344) may be moved out of engagement with collar portion (226) of axial locator (222).

Once retaining arms (340) are disengaged from axial locator (222), cutter carriage (320) may be axially released relative to latch mechanism (220), thereby permitting free axial translation of cutter carriage (320). Cutter spring (312) may then be free to decompress, thereby driving cutter carriage (320) distally to the fired configuration shown in FIG. 19. As cutter carriage (320) is moved to the fired configuration, cutter (40) may likewise move distally relative to piercer (22), thereby severing a tissue sample into notch (26).

After drive assembly (200) is moved to the fired configuration, the severed tissue sample may be collected by returning drive assembly (200) to the initial position described above and subsequently rotating control shaft (360) to wipe the severed tissue sample from notch (26). The same process may then be repeated to collect any number of desired tissue samples with a single insertion of needle assembly (20).

### B. Example Alternative Drive Assembly with Axially Aligned Drives

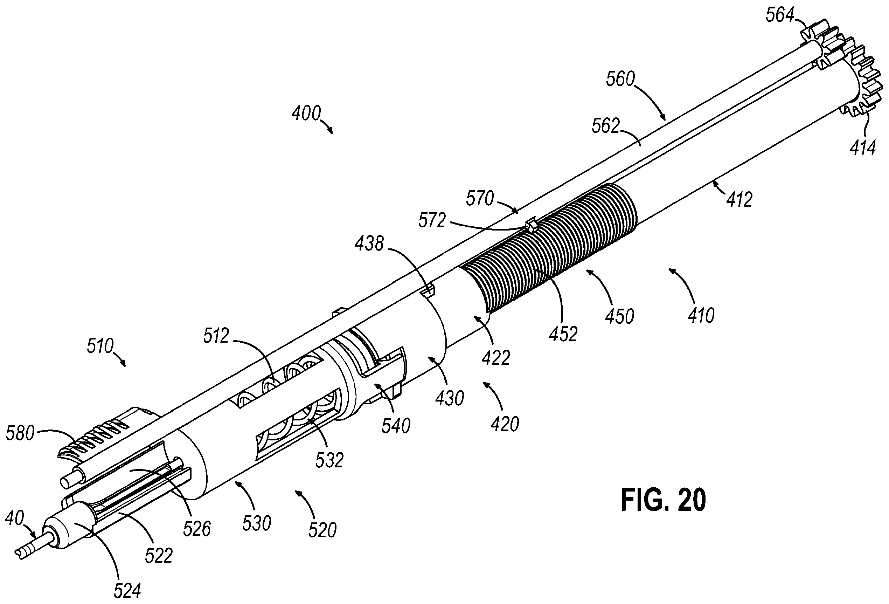

FIG. 20 shows a drive assembly (400) that may be readily incorporated into biopsy device (10) described above in lieu of drive assembly (200) described above. Similar to drive assembly (200) described above, drive assembly (400) of the present version may be generally configured to manipulate portions of needle assembly (20) such as piercer (22) and cutter (40). In some versions, this manipulation may include driving piercer (22) and/or cutter (40) through a predetermined sequence for cocking, firing, and sample collection.

To facilitate such sequential operation, drive assembly (400) includes a piercer drive assembly (410) and a cutter drive assembly (510). As will be described in greater detail below, piercer drive assembly (410) and cutter drive assembly (510) are generally interconnected with each other and generally configured to interact with each other. As a result of this interconnected configuration, piercer drive assembly (410) and cutter drive assembly (510) may operate to move piercer (22) and cutter (40) independently, while also in a predetermined sequence in a relatively compact size across the axial dimension.

Piecer drive assembly (410) may be in communication with piercer (22) such that piercer drive assembly (410) may be configured to drive piercer (22) through a predetermined sequence of movement independently from cutter (40), in concert with cutter (40), or both. Additionally, as will be described in greater detail below, piercer drive assembly (410) may be in communication with one or more elements of cutter drive assembly (510) to drive elements of cutter drive assembly (510) and/or cutter (40).

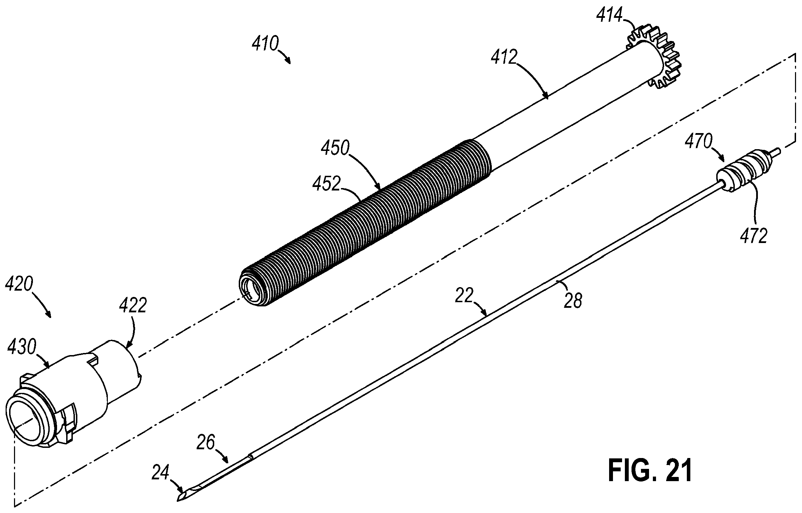

As best seen in FIG. 21, piercer drive assembly (410) includes a lead screw shaft (412), a latch mechanism (420), and a piercer carriage (470). Additionally, lead screw shaft (412) defines an internal piercer lead screw (440) (also referred to as primary lead screw, or a piercer driver), an external cutter lead screw (450) (also referred to as a secondary lead screw, or a cutter driver). Generally, piercer drive assembly (410) is configured to move piercer (22) via piercer carriage (470) by moving piercer carriage (470) directly via rotation of lead screw shaft (412).

Lead screw shaft (412) is generally configured to drive rotation of piercer lead screw (440) and cutter lead screw (450). Lead screw shaft (212) extends axially along a longitudinal axis defined by needle assembly (20) such that piercer lead screw (440) and cutter lead screw (450) are both positioned along the longitudinal axis defined by needle assembly (20). Lead screw shaft (412) further includes a drive gear (414) that may be fastened thereto or integral therewith, to drive rotation of lead screw shaft (412). Thus, drive gear (414) may be configured to drive rotation of piercer lead screw (440) via lead screw shaft (412). Although not shown, it should be understood that drive gear (414) may mesh with other components of biopsy device (10) such as a motorized assembly to drive rotation of lead screw shaft (412).

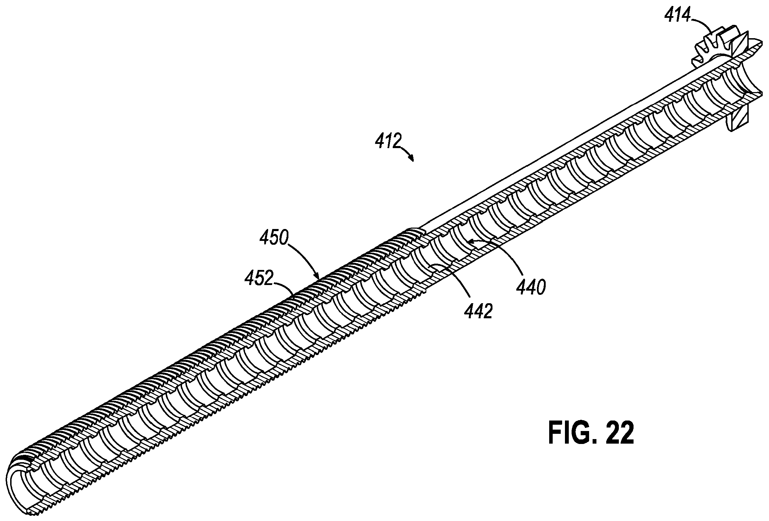

As best seen in FIG. 22, piercer lead screw (440) extends internally within a hollow interior defined by lead screw shaft (412). Piercer lead screw (440) is generally cylindrical in shape and includes threading (442). Although not shown, it should be understood that piercer lead screw (440) may optionally include a hard stop (not shown) at a proximal end of threading (442) to facilitate operational functions such as initialization. As will be understood, threading (442) may be generally configured to engage piercer carriage (470) to convert rotation of piercer lead screw (440) into translation of piercer (22) via piercer carriage (470). Similarly, the hard stop may be configured to engage a portion of piercer carriage (470) to provide a mechanical stop to translation of piercer carriage (470) proximate the proximal end of piercer lead screw (440).

Threading (442) in the present version is generally coarse such that rotation of piercer lead screw (440) may result in greater translation of piercer carriage (470) relative to other forms of threading described herein. Threading (442) of the present version is also of a right-handed configuration such that clockwise rotation (as viewed from the proximal end of piercer lead screw (440) looking distally) of piercer lead screw (440) may result in distal translation of piercer carriage (470). Although right-handed threading is used in the present version, it should be understood that in other versions this threading may be reversed. However, as will be described in greater detail below, such a reversal of threading (442) may result in corresponding reversal of other forms of threading associated with other components described herein.

Cutter lead screw (450) extends externally along the outer surface of lead screw shaft (412). In this configuration, at least a portion of cutter lead screw (450) may optionally overlap with piercer lead screw (440). Cutter lead screw (450) also defines a generally cylindrical shape similar to the cylindrical shape of piercer lead screw (440). However, the diameter of cutter lead screw (450) may generally be greater than the diameter of piercer lead screw (440) due to the internal and external configuration of piercer lead screw (440) and cutter lead screw (450), respectively. It should be understood that cutter lead screw (450) may be fixedly secured or integral with piercer lead screw (440). Thus, rotation of piercer lead screw (440) may result in corresponding rotation of piercer lead screw (440).

Cutter lead screw (450) may also include threading (452). As will be understood, threading (452) of cutter lead screw (450) may be generally configured to engage latch mechanism (420) to convert rotation of cutter lead screw (450) into translation of latch mechanism (420) along the longitudinal axis of cutter lead screw (450).

Threading (452) of cutter lead screw (450) in the present version is generally fine relative to threading (442) of piercer lead screw (440). Thus, rotation of cutter lead screw (450) may generally result in less translation of latch mechanism (420) relative to translation of piercer carriage (470) generated by piercer lead screw (440). As a result of this configuration, the axial length of threading (452) along lead screw shaft (412) may be shorter relative to the axial length of threading (442) within lead screw shaft (412).

Threading (452) of the present version is also opposite threading (442) of piercer lead screw (440). In other words, threading (452) of cutter lead screw (450) may be of a left-handed configuration such that clockwise rotation (as viewed from the proximal end of piercer lead screw (440) looking distally) of cutter lead screw (450) may result in proximal translation of latch mechanism (420). As will be understood, this opposing thread configuration may be desirable to promote certain sequential movements of piercer (22) and cutter (40). Although left-handed threading is used in the present version, it should be understood that in other versions, this threading may be reversed. However, as will be appreciated, such a reversal of threading (452) may result in corresponding reversal of threading (442) to still provide the opposing thread configuration described herein.

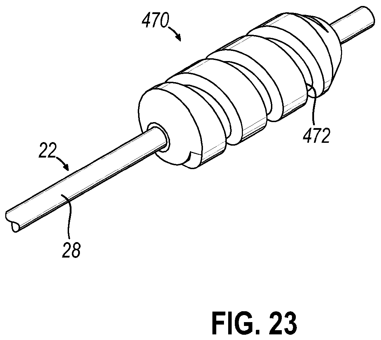

FIG. 23 shows piercer carriage (470) in greater detail. As can be seen, piercer carriage (470) is generally formed by an overmold onto a portion of piercer (22) proximate the proximal end thereof. Piercer carriage (470) is generally configured for receipt within the hollow interior of lead screw shaft (412) for engagement with piercer lead screw (440). Accordingly, piercer carriage (470) may include threading (472) on an outer surface thereof for engagement with threading (442) of piercer lead screw (440).

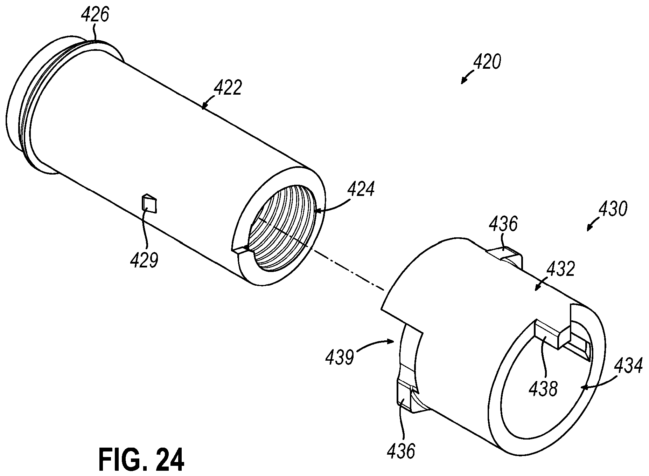

FIGS. 24 through 26 show latch mechanism (420) in greater detail. Although latch mechanism (420) is characterized herein as being a part of piercer drive assembly (410), latch mechanism (420) may also be characterized as being a part of cutter drive assembly (510), another portion of drive assembly (400), or a separate component entirely. As will be understood, latch mechanism (420) is generally configured to interact with various components of drive assembly (400) to facilitate interaction between piercer drive assembly (410) and cutter drive assembly (510).

Latch mechanism (420) includes an axial locator (422) (also referred to as a nut member) and a cam member (430). As will be described in greater detail below, axial locator (422) and cam member (430) are generally configured to work cooperatively to move along the longitudinal axis of cutter lead screw (450) and selectively engage and/or disengage one or more portions of cutter drive assembly (510).

As best seen in FIG. 24, axial locator (422) defines a generally cylindrical shape. Axial locator (422) defines a threaded bore (424), a collar portion (426), and a receiving portion (428). Threaded bore (424) may extend longitudinally through axial locator (422) and may include threading (not shown) configured to engage threading (452) of cutter lead screw (450). Thus, it should be understood that axial locator (422) may be configured to axially translate along the length of cutter lead screw (450) in response to rotation of cutter lead screw (450).

Both collar portion (426) and receiving portion (428) are configured to work cooperatively to receive and locate cam member (430) relative to axial locator (422). Collar portion (426) extends outwardly from receiving portion (428) defining a structure similar to a flange. As will be described in greater detail below, collar portion (426) is generally configured to engage one or more portions of cutter drive assembly (510) to associate movement of latch mechanism (420) with cutter drive assembly (510). Optionally, a distal portion of collar portion (426) may be sloped, angled, or rounded to promote engagement with cutter drive assembly (510).

Receiving portion (428) defines a generally smooth cylindrical surface configured for receipt within a portion of cam member (430), as will be described in greater detail below. Optionally, receiving portion (428) may include an alignment protrusion (429). Alignment protrusion (429) may be configured to retain cam member (430) on axial locator (422) between collar portion (426) and alignment protrusion (429). In some versions, a proximal surface of alignment protrusion (429) may be sloped to facilitate ease of assembly. Additionally, although a single alignment protrusion (429) is shown, in other versions, any suitable numbers of alignment protrusions (429) may be used.

Cam member (430) is generally configured to receive axial locator (422) and to rotate relative to axial locator (422). Cam member (430) includes a body (432) defining a receiving bore (434), a one or more cam features (436) projecting from an outer surface of body (432), and one or more axial projections (438) extending axially from body (432). Receiving bore (434) is generally configured to receive at least a portion of axial locator (422), while cam features (436), and axial projections (438) are generally configured to promote manipulation of cam member (430) relative to axial locator (422) for engagement with one or more portions of cutter drive assembly (510).

Cam member (430) of the present version includes a pair of cam features (436), although other suitable numbers may be used in other versions. Each cam feature (436) is generally configured to engage a portion of cutter drive assembly (510) upon rotation of cam member (430) as will be described in greater detail below. To facilitate such engagement, a portion of each cam member (430) may be sloped or contoured. On other words, each cam feature (436) may include a camming surface to drive movement of one or more portions of cutter drive assembly (510).

Cam member (430) of the present version includes a single axial projection (438) extending proximally as opposed to the pair of axial projections (238) discussed above with respect to cam member (240). Alternatively, in other versions, axial projection (438) may be configured with multiple projections. Additionally, each axial projection (438) may extend proximally or distally.

Axial projection (438) may be disposed between each cam feature (436) about the circumference of cam member (430). As a result of the proximal extension of axial projection (438), axial projection (438) extends into an open space proximate body (432). Such an open space may permit extension of one or more portions of cutter drive assembly (410) relative to cam member (430) or other parts of drive assembly (400) to engage axial projection (438).

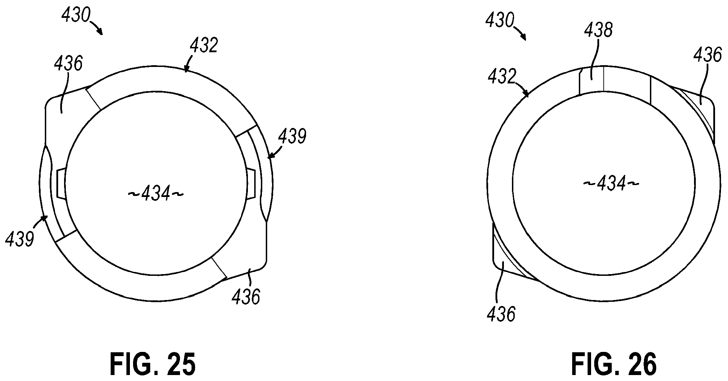

As best seen in FIGS. 25 and 26, body (432) further defines a pair of pockets (439) extending axially from a distal edge of body (432). Pockets (439) are generally configured to permit a portion of cutter drive assembly (510) to extend into a portion of body (432) in a region of body (432) proximate cam features (436). As will be understood, this configuration may promote engagement between portions of cutter drive assembly (510) and cam features (436) upon rotation of cam member (430).

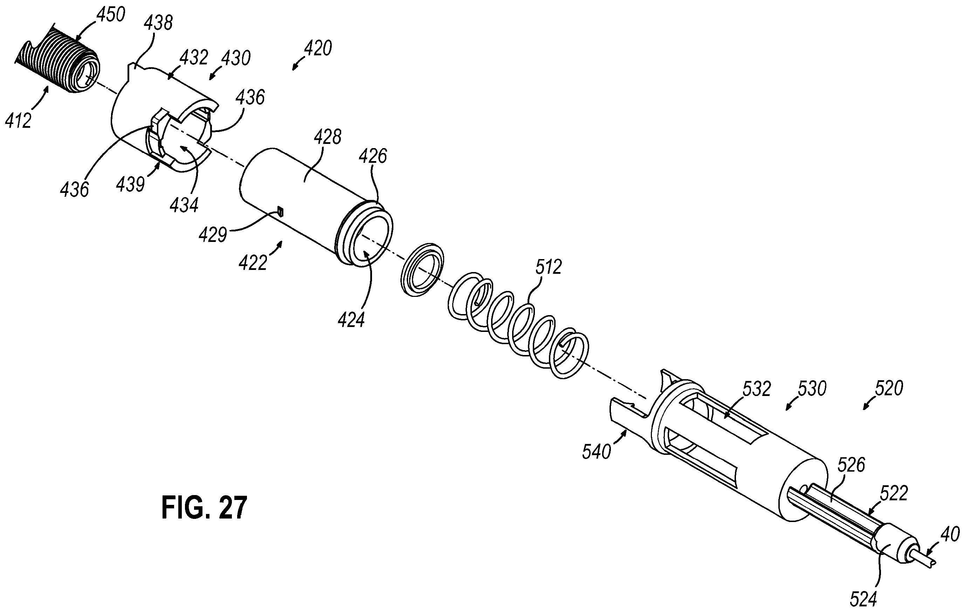

FIG. 27 shows cutter drive assembly (510) in greater detail. As can be seen, cutter drive assembly (510) includes a cutter spring (512) and a cutter carriage (520). Additionally, it should be understood that in some contexts, latch mechanism (420) may be characterized as a part of cutter drive assembly (510) rather than piercer drive assembly (410) because latch mechanism (420) may engage with elements of both assemblies (410, 510). As will be understood, cutter drive assembly (510) is generally configured to engage with piercer drive assembly (410) and other elements of drive assembly (400) to sequentially actuate and fire cutter (40).

Cutter carriage (520) includes a carriage body (522) that defines a cutter collar (524), tissue manipulator (526), and a proximal receiving end (530). Cutter collar (524) is configured to receive the proximal end of cutter (40) such that cutter (40) may extend distally from cutter collar (524). In the present version, cutter (40) may be fixedly secured to cutter collar (524). Alternatively, in other versions, cutter collar (524) may be threaded, keyed, or otherwise structured to permit cutter (40) to be removably secured to cutter collar (524). Cutter collar (524) is generally of a hollow configuration to promote access to the proximal end of cutter (40) by piercer (22) and/or other structures.

Tissue manipulator (526) is disposed between cutter collar (524) and proximal receiving end (530). Tissue manipulator (526) is generally configured to direct tissue from piercer (22) into a tissue sample chamber or other structure during operation of drive assembly (400). Thus, tissue manipulator (526) is configured to receive at least a portion of piercer (22) such that piercer (22) may extend and move through tissue manipulator (526). In some versions, tissue manipulator (526) may be used in connection with other features such as a flexible member, a wiper, or a blade feature. In such versions, tissue manipulator (536) and associated features may be configured in accordance with one or more teachings of U.S. Ser. No. 63/316,184, entitled "Sample Management for Core Needle Biopsy Device," filed on March 3, 2022.

Proximal receiving end (530) of carriage body (522) is generally configured to engage various components of drive assembly (400) to drive movement of cutter (40) via movement of cutter carriage (520). In particular, proximal receiving end (530) defines a spring bore (532) aligned with a longitudinal axis defined by needle assembly (20). Spring bore (532) may be configured to receive cutter spring (512) such that cutter spring (512) may be compressed within spring bore (532) between a portion of cutter carriage (520) and latch mechanism (420), as will be described in greater detail below. Although spring bore (532) is shown in the present version as defining generally cylindrical passageway through a portion of cutter carriage (520), it should be understood that in other versions, spring bore (532) may use various shapes or may be omitted entirely.

Proximal receiving end (530) further includes one or more retaining arms (540) extending proximally from a proximal surface of proximal receiving end (530). Retaining arms (540) are generally configured to resiliently engage a portion of latch mechanism (420) to permit selective coupling of cutter drive assembly (510) to piercer drive assembly (410), as will be described in greater detail below. Each retaining arm (540) includes a respective extension member (542) and engagement member (544). Each extension member (542) is generally configured to have some rigidity, but also some flexibility. As will be described in greater detail below, this combination of rigidity and flexibility may be desirable to promote selective coupling and decoupling with respect to latch mechanism (420).

Each engagement member (544) is generally configured as a tooth or catch. As will be understood, this configuration may permit each engagement member (544) to engage collar portion (426) of axial locator (422). Specifically, each engagement member (544) includes a flat face (546) and an angled surface (548) projecting proximally relative to flat face (546). As will be described in greater detail below, flat face (546) may be configured to engage collar portion (426) of axial locator (422) for fastening, while angled surface (548) may be configured to engage collar portion (426) for manipulation of each respective retaining arm (540) during fastening.

In the present configuration, proximal receiving end (530) includes a pair of retaining arms (540) oriented opposite each other around the perimeter of a circle corresponding to about the diameter of axial locator (422). In other versions, any suitable number of retaining arms (540) may be used such as three, four, or more. Alternatively, in still other versions, retaining arms (540) may be configured as a single element with relief slots formed in a structure thereof to promote similar functionality in a single element component.

Cutter spring (512) is configured to engage proximal receiving end (530) to drive cutter carriage (520) distally during firing of cutter (40). Specifically, cutter spring (512) includes a coil spring configured to be disposed between a portion of proximal receiving end (530), retaining arms (540) and latch mechanism (520). As will be described in greater detail below, cutter spring (512) is configured to be compressed between proximal receiving end (530) and latch mechanism (420) to store potential energy that may be released during firing of cutter (40).

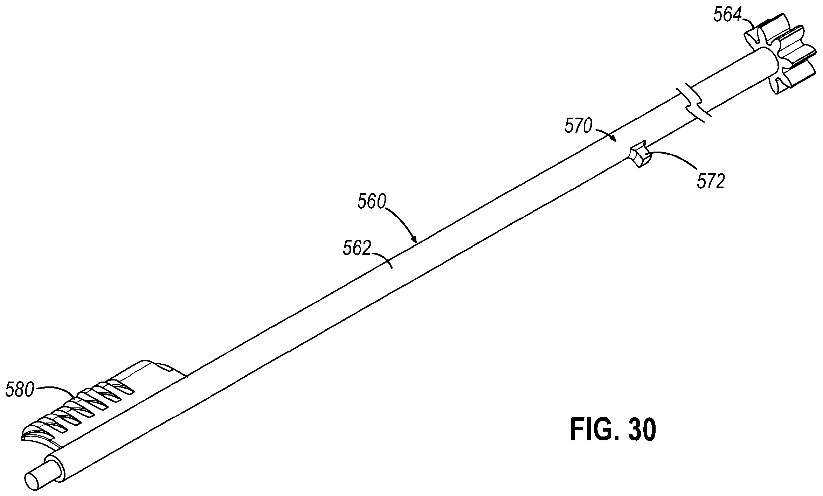

Drive assembly (400) further includes a control shaft (560) (also referred to as an actuation mechanism, control, or trip mechanism), which may be configured to interact with cutter drive assembly (510) to initiate movement of cutter (40) through a predetermined sequence of motion. As best seen in FIG. 30, control shaft (560) includes an elongate shaft (562) having a drive gear (564), a latch portion (570), and a tissue manipulation portion (580). Elongate shaft (562) defines a generally cylindrical cross-section and is configured to extend from piercer drive assembly (410) to cutter drive assembly (510).

Drive gear (564) is configured to engage a gear associated with a motor or other drive mechanism to drive rotation of control shaft (560). In some versions, drive gear (564) may be driven by the same motor used to drive gear (414) of piercer drive assembly (410) using a transmission, gearing, and/or other mechanisms. In other versions, drive gear (564) may be driven by a dedicated motor. In still other versions, drive gear (564) may be driven manually by a knob, thumbwheel, crank, or other manual drive mechanism.

Latch portion (570) is disposed along elongate shaft (562) between drive gear (564) tissue manipulation portion (580). Latch portion (570) is generally configured to engage a portion of latch mechanism (420) to control one or more operations of cutter drive assembly (510). In particular, latch portion (570) includes an actuator (572) protruding outwardly from elongate shaft (562). As will be described in greater detail below, actuator (572) is configured to engage one or more of axial projections (438) of cam member (430) to selectively release cutter carriage (520) from latch mechanism (420).

Tissue manipulation portion (580) is disposed proximate the distal end of elongate shaft (562). Tissue manipulation portion (580) is generally configured to manipulate a tissue sample out of piercer (22) and into a tissue chamber in response to rotation of control shaft (560). Tissue manipulation portion (580) may include a variety of features to facilitate such functionality. In some versions, tissue manipulation portion (580) may be configured in accordance with one or more teachings of U.S. Ser. No. 63/316,184, entitled "Sample Management for Core Needle Biopsy Device," filed on March 3, 2022.

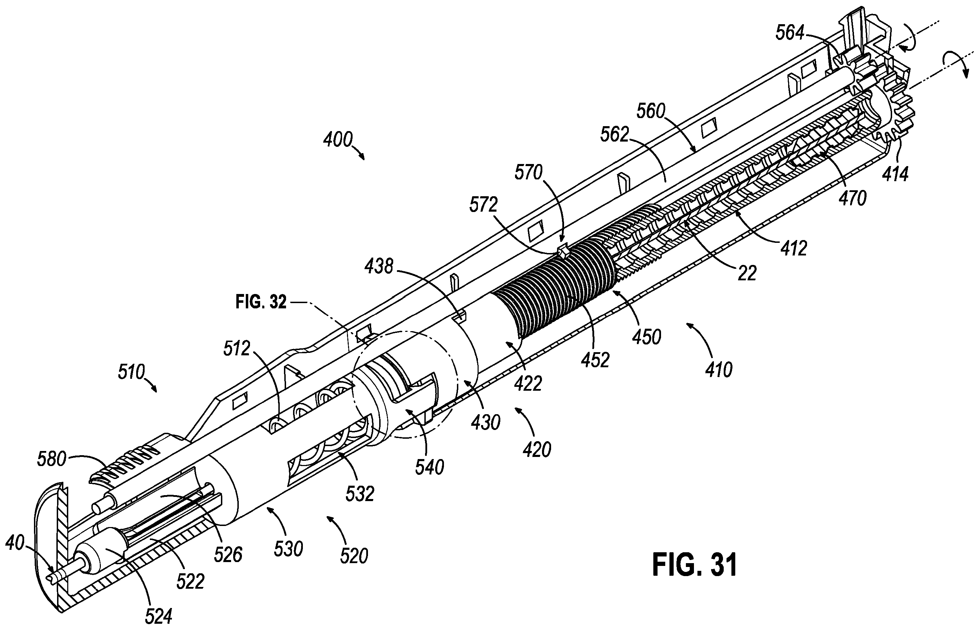

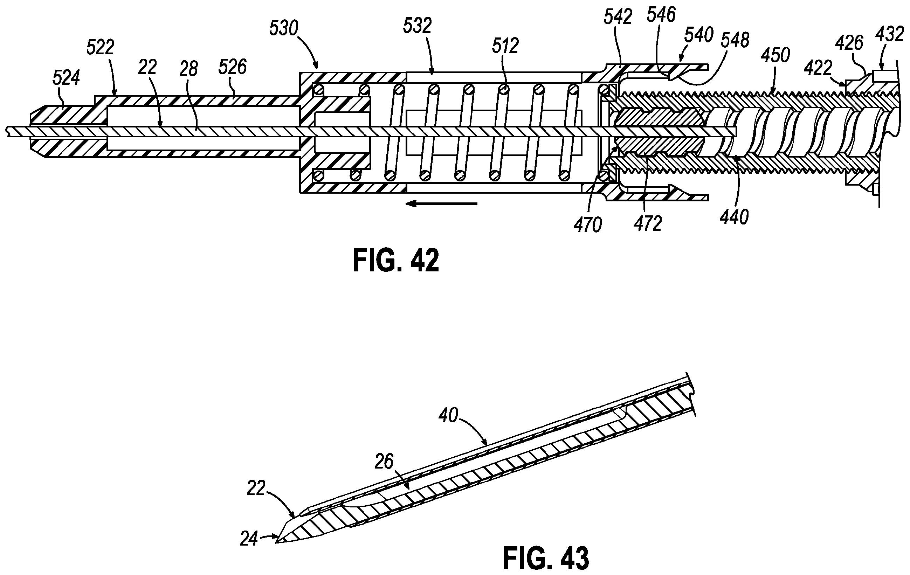

FIGS. 31 through 43 show an example use of drive assembly (400) in connection with biopsy device (10) to collect one or more tissue samples using a single insertion of needle assembly (20). As best seen in FIG. 31, drive assembly (400) may initially begin in an initial configuration (also referred to as a home or retrieve configuration). To transition to the initial configuration, lead screw shaft (412) may be rotated to drive piercer carriage (470) proximally via piercer lead screw (440) and threading (442). The same rotation of lead screw shaft (412) may also drive distal translation of latch mechanism (420) along cutter lead screw (450) via threading (452), which may be opposite in pitch relative to threading (442).

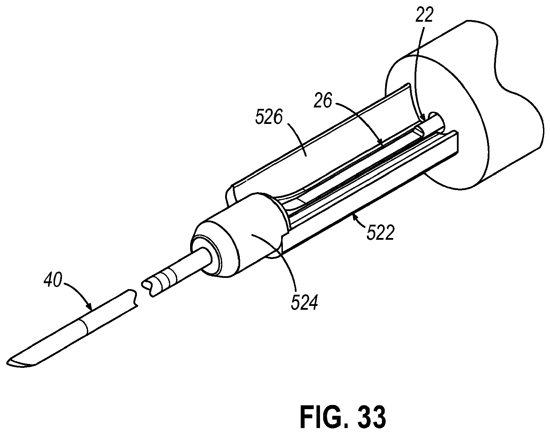

Once rotation of lead screw shaft (412) is completed to position drive assembly (400) in the initial position, piercer carriage (470) may be fully retracted proximally relative to piercer lead screw (440). In this position, piercer (22) may be in a proximal-most position. In such a position, notch (26) may be aligned tissue manipulator (526) of cutter carriage (520), as shown in FIG. 33. Thus, although the initial configuration may be used as a start or home position, the initial configuration may additionally be used as a sample collection position, as will be described in greater detail below.

Also in the initial position, cutter carriage (520) may be fully advanced distally relative to cutter lead screw (450). Specifically, latch mechanism (420) may advance distally via cutter lead screw (450), engage cutter carriage (520), and drive cutter carriage (520) distally to the initial position shown in FIG. 31. In this position, cutter (40) may be in a distal-most position (also referred to as a fired position). In some versions, cutter carriage (520) may abut a distal hard stop within the outer housing of biopsy device (10) to prevent further distal movement of cutter (40).

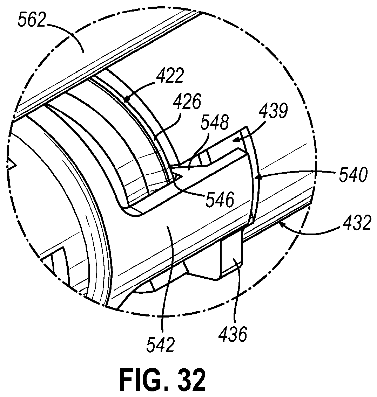

As described above, latch mechanism (420) may also be fully advanced distally via cutter lead screw (450). During distal advancement of latch mechanism (420), latch mechanism (420) may engage cutter carriage (520). As best seen in FIG. 32, latch mechanism (520) may contact retaining arms (540) of cutter carriage (520). As latch mechanism (420) is advanced further distally, collar portion (426) of axial locator (422) may engage angled surface (548) of each retaining arm (540), driving retaining arms (540) away from each other until collar portion (426) is disposed distally of flat face (546). Each flat face (546) may then engage collar portion (426) of axial locator (422), thereby coupling latch mechanism (420) to cutter carriage (520).

It should be understood that in some versions, coupling of latch mechanism (420) to cutter carriage (520) may also simultaneously compress cutter spring (512). In such uses, this may result in cutter (40) being characterized as partially armed. When partially armed, cutter spring (512) may be fully compressed in preparation for firing of cutter (40). However, due to the distal position of cutter carriage (520), actual firing of cutter (40) may be blocked or otherwise prevented.

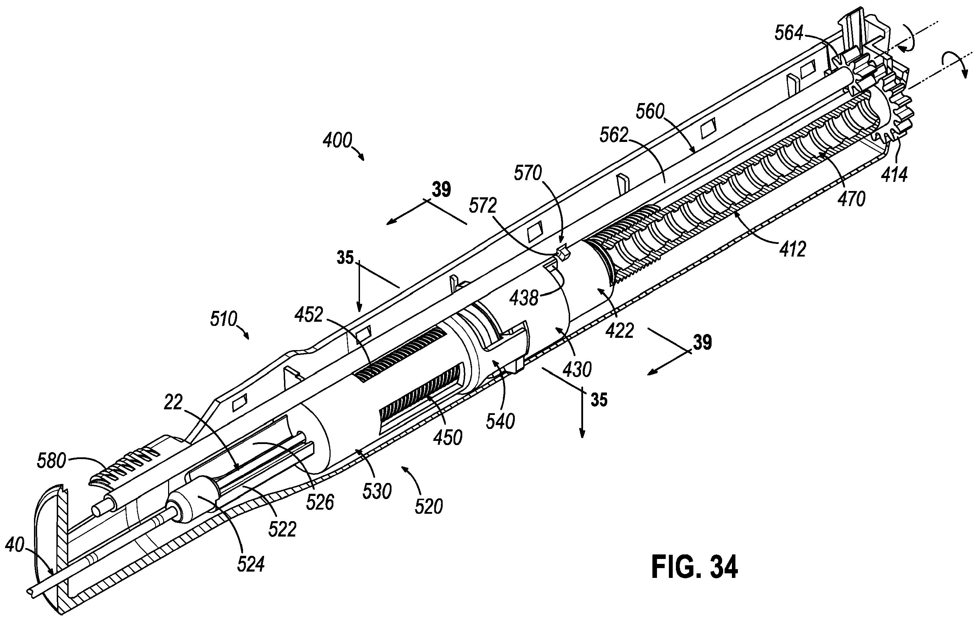

Once drive assembly (400) is in the initial configuration, it may be desirable to initiate arming of drive assembly (400). FIG. 34 shows drive assembly (400) in the armed configuration. In the present version, the transition from the initial configuration to the armed configuration may be initiated by an operator providing a user input (e.g., pressing a button on biopsy device (10)). Alternatively, in some versions, the transition to the armed configuration may be initiated automatically.

Regardless of the particular initiation process, the transition from the initial configuration to the armed configuration may begin by lead screw shaft (412) rotating in an opposite direction relative to the direction described above to translate piercer carriage (470) distally via piercer lead screw (440). Simultaneously, cutter lead screw (450) may rotate in the same direction as piercer lead screw (440) to translate latch mechanism (420) and cutter carriage (520) proximally. In other words, piercer carriage (470) and cutter carriage (520) may translate in opposite directions via the opposite hand threading of piercer lead screw (440) and cutter lead screw (450).

Rotation of piercer lead screw (440) and cutter lead screw (450) via lead screw shaft (412) may continue until piercer carriage (470) reaches the position shown in FIG. 34 and cutter carriage (520) reaches the proximal position also shown in FIG. 34. In some versions, piercer carriage (470) may reach its distal-most position and cutter carriage (520) may reach its proximal-most position at this stage.

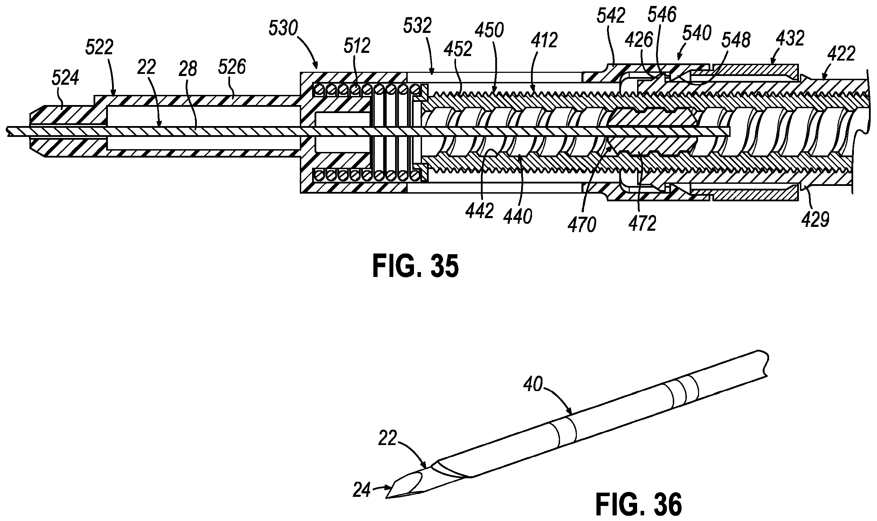

In other versions, piercer carriage (470) and cutter carriage (520) may optionally stop translation short of their respective distal and proximal-most positions to position piercer (22) and cutter (40) in a short stop configuration. For instance, as shown in FIG. 35, piercer carriage (470) may be partially advanced distally, short of its distal-most position. Similarly, cutter carriage (520) may be partially retracted proximally, short of its proximal-most position. As a result of this short stop configuration, piercer (22) may be positioned relative to cutter (40) as shown in FIG. 36. In this configuration, piercer (22) is partially extended from cutter (40) with distal tip (24) protruding from cutter (40), while notch (26) may remain within cutter (40). It may be desirable to use this short stop configuration for insertion of needle assembly (20) into tissue because notch (26) being disposed within cutter (40) may reduce the force required to penetrate tissue. It should be understood that in some versions, drive assembly (400) may stop automatically upon transitioning to the short stop configuration. After this point, further user input (e.g., a second press of a button) may be used to initiate further rotation of lead screw shaft (412). In some versions, a series of light emitting diodes (LED) may be used to indicate a status of drive assembly (400) (e.g., one LED for armed, two LED for exposed notch (26), three LED for fired).

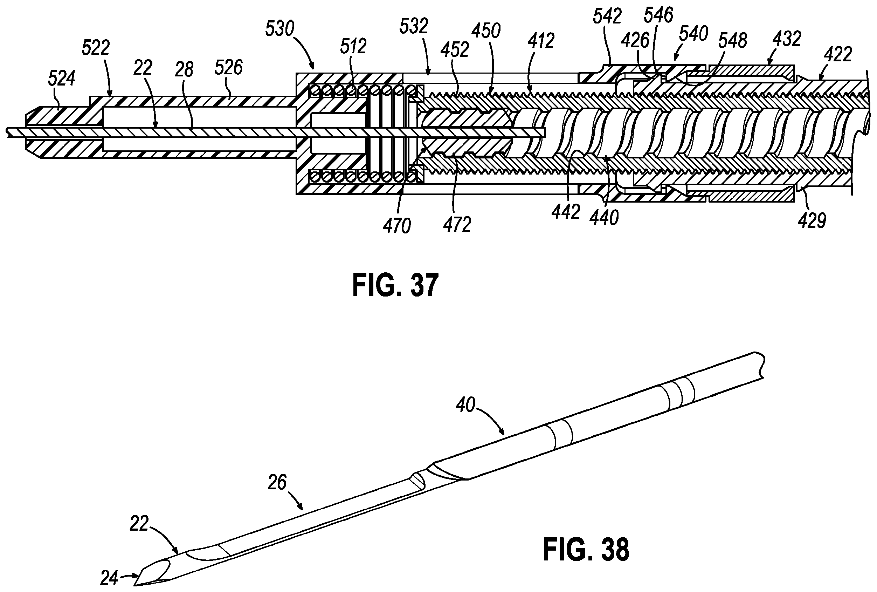

After translating piercer carriage (470) and cutter carriage (520) to the short stop configuration described above, rotation of lead screw shaft (412) may continue with piercer carriage (470) to the distal-most position and cutter carriage (520) translating to the proximal-most position as shown in FIG. 37. With piercer carriage (470) in the distal position, piercer (22) may be in a distal-most position. Similarly, with cutter carriage (320) in the proximal position, cutter (40) may be in a proximal-most position. This may result in a relative position between piercer (22) and cutter (40) that includes notch (26) of piercer (22) being exposed relative to cutter (40) as shown in FIG. 38.

Retraction of cutter carriage (520) proximally via engagement with latch mechanism (420) may also serve to arm or cock cutter drive assembly (510) for firing. Specifically, in versions where cutter spring (512) is compressed during the transition to the initial configuration, arming or cocking may include retracting cutter carriage (520) proximally to provide space for subsequent distal advancement of cutter carriage (520) during firing of cutter (40).

In other versions, proximal translation of cutter carriage (520) may also be used for compression of cutter spring (512). Specifically, in some versions, cutter spring (512) may be in another position relative to cutter carriage (520) rather than being compressed between cutter carriage (520) and latch mechanism (420). By way of example only, in some versions, cutter spring (512) may be compressed between cutter carriage (520) and a portion of a housing of biopsy device (10). In such versions, compression of cutter spring (512) may be based on movement of cutter carriage (520) relative to the housing rather than the movement of cutter carriage (520) relative to latch mechanism (420). Thus, in such versions, proximal translation of cutter carriage (520) during arming or cocking may also include compression of cutter spring (512).

Once drive assembly (400) is moved into the armed configuration shown in FIG. 34, piercer (22) and cutter (40) may be inserted into tissue and positioned proximate a lesion. Such insertion into tissue may be performed either with notch (26) of piercer (22) exposed relative to cutter (40) or notch (26) covered by cutter (40) as described above. Optionally, insertion at this stage may be performed in combination with various forms of image guidance such as ultrasonic, x-ray, magnetic resonance imaging (MRI), and/or etc.

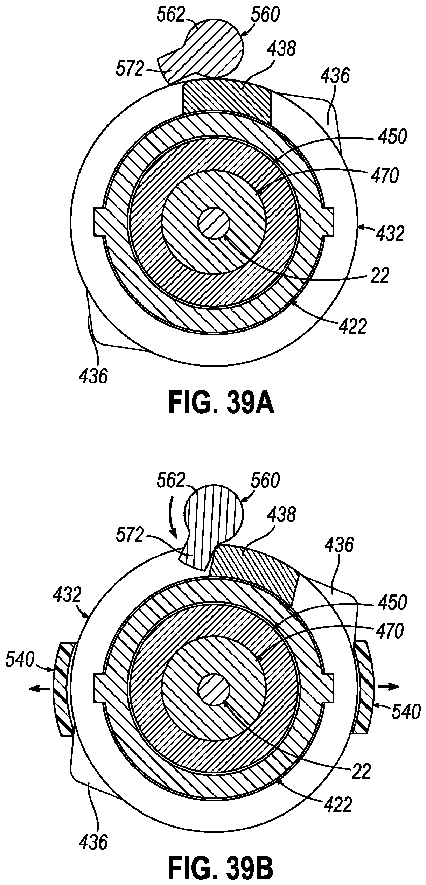

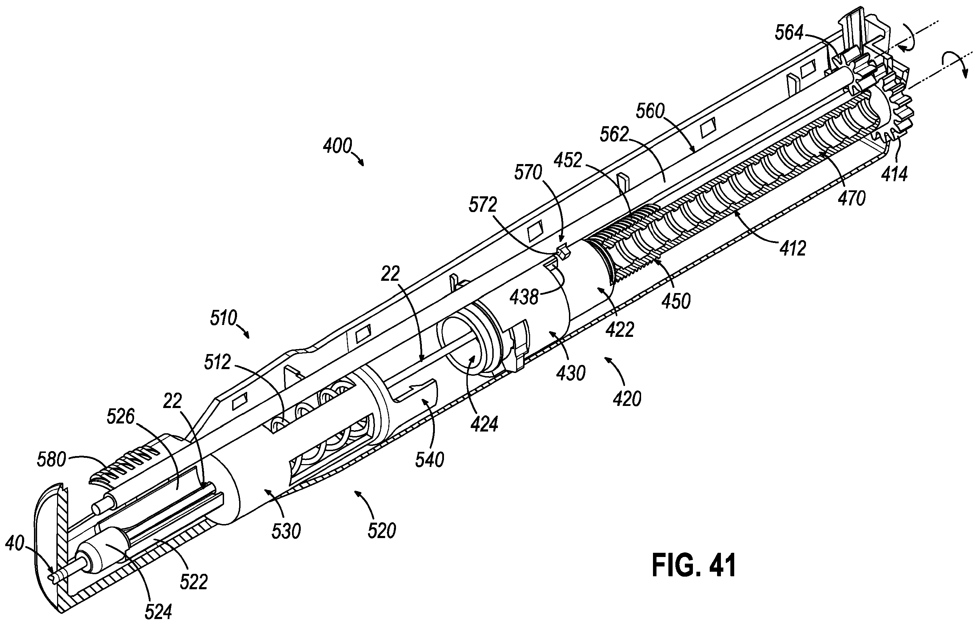

After drive assembly (400) is moved to the armed configuration, it may be desirable to fire cutter (40) relative to piercer (22) to sever a tissue sample into notch (26) of piercer (22). As similarly discussed above for the armed configuration, firing may be initiated using a user input such as a button. FIGS. 39A through 40B show a sequence that may occur to move drive assembly (400) into a fired configuration shown in FIG. 41.

As best seen in FIGS. 39A through 39B, the firing sequence may begin with control shaft (560) rotating to move actuator (572) of latch portion (570) toward latch mechanism (420). At this stage, actuator (572) may engage axial projections (438) or other suitable portions of cam member (430) to rotate cam member (430) relative to axial locator (422). As cam member (430) is rotated, cam features (436) may be driven into engagement with retaining arms (540) of cutter carriage (520).

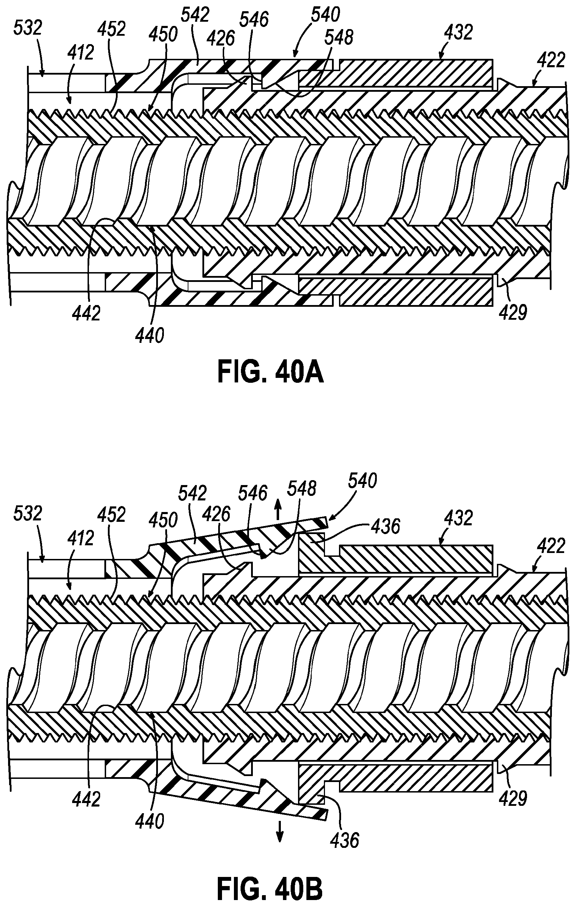

Further rotation of cam member (430) may cause retaining arms (540) of cutter carriage (520) to release from collar portion (426) of axial locator (422). Specifically, as best seen in FIGS. 40A through 40B, rotation of cam member (430) may cause cam features (436) to exert outward pressure on each retaining arm (540) of cutter carriage (520). As a result, flat face (546) of engagement member (544) may be moved out of engagement with collar portion (426) of axial locator (422).

Once retaining arms (540) are disengaged from axial locator (422), cutter carriage (520) may axially released relative to latch mechanism (420), thereby permitting free axial translation of cutter carriage (520). Cutter spring (512) may then be free to decompress, thereby driving cutter carriage (520) distally to the fired configuration shown in FIGS. 41 through 43. As cutter carriage (520) is moved to the fired configuration, cutter (40) may likewise move distally relative to piercer (22), thereby severing a tissue sample into notch (26).

After drive assembly (400) is moved to the fired configuration, the severed tissue sample may be collected by returning drive assembly (400) to the initial position described above and subsequently rotating control shaft (560) to wipe the severed tissue sample from notch (26). The same process may then be repeated to collect any number of desired tissue samples with a single insertion of needle assembly (20).

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A core needle biopsy device, comprising:
(a) a needle assembly (20), the needle assembly (20) including a piercer (22) and a hollow cutter (40), the piercer (22) including a sharp distal tip (24) and a notch (26) proximate the distal tip (24), the piercer (22) being slidably disposed within the cutter (40) to sever a tissue sample into the notch of the piercer (22);
(b) a cutter drive assembly (310, 510) configured to move the cutter (40);
(c) a piercer drive assembly (210, 410) configured to move the piercer (22), the piercer drive assembly (310, 410) including a lead screw, the lead screw being configured to move both a portion of the cutter drive assembly (310, 510) and a portion of the piercer drive assembly (210, 410); and
(d) a latch mechanism (220, 420) configured to selectively couple a portion of the cutter drive assembly (310, 510) to a portion of the piercer drive assembly (210, 410)
the lead screw including a piercer lead screw (240, 440) including a first threading (242, 442) and a cutter lead screw (250, 450) including a second threading (252, 452), the piercer lead screw (240, 440) being configured to drive translation of the piercer (22), the cutter lead screw (250, 450) being configured to drive translation of the cutter (40),
**characterised in that** the second threading (252, 452) being an opposite hand threading to the first threading (242, 442) such that rotation of the lead screw in a single direction drives translation of the piercer (22) and cutter (40) in opposite directions.

2. The core needle biopsy device of claim 1, the first threading (242, 442) having a coarse pitch relative to the second threading (252, 452).

3. The core needle biopsy device of claim 2, the piercer lead screw (240) and cutter lead screw (250) being both external relative to a structure of the lead screw.

4. The core needle biopsy device of any of claims 2 through 3, the piercer lead screw (440) or the cutter lead screw (450) being internal relative to a structure of the lead screw.

5. The core needle biopsy device of any of claims 1 through 4, the lead screw being oriented along an axis offset relative to a longitudinal axis defined by the needle assembly (20).

6. The core needle biopsy device of any of claims 1 through 4, the lead screw being oriented along a longitudinal axis defined by the needle assembly (20).

7. The core needle biopsy device of any of claims 1 through 6, the cutter drive assembly (310, 510), the piercer drive assembly (210, 410), and the latch mechanism (220, 420) being oriented along a single axis, the latch mechanism (220, 420) being disposed between a portion of the cutter drive assembly (310, 510) and the piercer drive assembly (210, 410).

8. The core needle biopsy device of any of claims 1 through 7, the latch mechanism (220, 420) including an axial locator (222, 422) and a cam member (230, 430), the cam member (230, 430) being configured to rotate relative to the axial locator (222, 422) to selectively decouple a portion of the cutter drive assembly (310, 510) from a portion of the piercer drive assembly (210, 410).

9. The core needle biopsy device of claim 8, the cam member (230, 430) of the latch mechanism (220, 420) including one or more cam features (236, 436), the one or more cam features (236, 436) being configured to manipulate a portion of the cutter drive assembly (310, 510) upon rotation of the cam member (230, 430) relative to the axial locator (222, 422).

10. The core needle biopsy device of claims 8 or 9, further comprising a control shaft (360, 560), the control shaft (360, 560) including an actuator (372, 572), the actuator (372, 572) being configured to engage the cam member (230, 430) of the latch mechanism (220, 420) to rotate the latch mechanism (220, 420) relative to the axial locator (222, 422).

11. The core needle biopsy device of any of claims 1 through 9, further comprising a control shaft (360, 560), the control shaft (360, 560) being configured to rotate relative to the latch mechanism (220, 420) to engage the latch mechanism (220, 420) and selectively decouple a portion of the cutter drive assembly (310, 510) from the piercer drive assembly (210, 410).

12. The core needle biopsy device of any of claims 1 through 11, the lead screw being configured to drive axial translation of the latch mechanism (220, 420).

## Patentansprüche

1. Biopsievorrichtung für Kernnadel, umfassend:
(a) eine Nadelanordnung (20), wobei die Nadelanordnung (20) ein Durchstechelement (22) und eine hohle Schneidvorrichtung (40) einschließt, wobei das Durchstechelement (22) eine scharfe distale Spitze (24) und eine Aussparung (26) nahe der distalen Spitze (24) einschließt, wobei das Durchstechelement (22) innerhalb der Schneidvorrichtung (40) gleitend angeordnet ist, um eine Gewebeprobe in die Aussparung des Durchstechelements (22) abzutrennen;
(b) eine Schneidvorrichtungsantriebsanordnung (310, 510), die dazu ausgebildet ist, die Schneidvorrichtung (40) zu bewegen;
(c) eine Durchstechelementantriebsanordnung (210, 410), die dazu ausgebildet ist, das Durchstechelement (22) zu bewegen, wobei die Durchstechelementantriebsanordnung (310, 410) eine Gewindespindel einschließt, wobei die Gewindespindel dazu ausgebildet ist, sowohl einen Teil der Schneidvorrichtungsantriebsanordnung (310, 510) als auch einen Teil der Durchstechelementantriebsanordnung (210, 410) zu bewegen; und
(d) einen Rastmechanismus (220, 420), der dazu ausgebildet ist, einen Teil der Schneidvorrichtungsantriebsanordnung (310, 510) selektiv mit einem Teil der Durchstechelementantriebsanordnung (210, 410) zu koppeln
wobei die Gewindespindel eine Durchstechelementgewindespindel (240, 440), die ein erstes Gewinde (242, 442) einschließt, und eine Schneidvorrichtungsgewindespindel (250, 450) einschließt, die ein zweites Gewinde (252, 452) einschließt, wobei die Durchstechelementgewindespindel (240, 440) dazu ausgebildet ist, die Verschiebung des Durchstechelements (22) anzutreiben, wobei die Schneidvorrichtungsgewindespindel (250, 450) dazu ausgebildet ist, die Verschiebung der Schneidvorrichtung (40) anzutreiben,
**dadurch gekennzeichnet, dass** das zweite Gewinde (252, 452) ein gegenläufiges Gewinde zu dem ersten Gewinde (242, 442) ist, sodass eine Drehung der Gewindespindel in einer einzigen Richtung die Verschiebung des Durchstechelements (22) und der Schneidvorrichtung (40) in entgegengesetzten Richtungen antreibt.

2. Biopsievorrichtung für Kernnadel nach Anspruch 1, wobei das erste Gewinde (242, 442) eine gröbere Steigung relativ zu dem zweiten Gewinde (252, 452) aufweist.

3. Biopsievorrichtung für Kernnadel nach Anspruch 2, wobei die Durchstechelement-Gewindespindel (240) und die Schneidvorrichtungsgewindespindel (250) relativ zu einer Struktur der Gewindespindel beide außenliegend sind.

4. Biopsievorrichtung für Kernnadel nach einem der Ansprüche 2 bis 3, wobei die Durchstechelement-Gewindespindel (440) oder die Schneidvorrichtungsgewindespindel (450) relativ zu einer Struktur der Gewindespindel innenliegend ist.

5. Biopsievorrichtung für Kernnadel nach einem der Ansprüche 1 bis 4, wobei die Gewindespindel entlang einer gegenüber einer durch die Nadelanordnung (20) definierten Längsachse versetzten Achse ausgerichtet ist.

6. Biopsievorrichtung für Kernnadel nach einem der Ansprüche 1 bis 4, wobei die Gewindespindel entlang einer durch die Nadelanordnung (20) definierten Längsachse ausgerichtet ist.

7. Biopsievorrichtung für Kernnadel nach einem der Ansprüche 1 bis 6, wobei die Schneidvorrichtungsantriebsanordnung (310, 510), die Durchstechelementantriebsanordnung (210, 410) und der Rastmechanismus (220, 420) entlang einer einzigen Achse ausgerichtet sind, wobei der Rastmechanismus (220, 420) zwischen einem Teil der Schneidvorrichtungsantriebsanordnung (310, 510) und der Durchstechelementantriebsanordnung (210, 410) angeordnet ist.

8. Biopsievorrichtung für Kernnadel nach einem der Ansprüche 1 bis 7, wobei der Rastmechanismus (220, 420) ein Axialpositionierelement (222, 422) und ein Nockenelement (230, 430) einschließt, wobei das Nockenelement (230, 430) dazu ausgebildet ist, sich relativ zu dem Axialpositionierelement (222, 422) zu drehen, um einen Teil der Schneidvorrichtungsantriebsanordnung (310, 510) selektiv von einem Teil der Durchstechelementantriebsanordnung (210, 410) zu entkoppeln.

9. Biopsievorrichtung für Kernnadel nach Anspruch 8, wobei das Nockenelement (230, 430) des Rastmechanismus (220, 420) eine oder mehrere Nockenstrukturen (236, 436) einschließt, wobei die eine oder die mehreren Nockenstrukturen (236, 436) dazu ausgebildet sind, einen Teil der Schneidvorrichtungsantriebsanordnung (310, 510) bei Drehung des Nockenelements (230, 430) relativ zu dem Axialpositionierelement (222, 422) zu betätigen.

10. Biopsievorrichtung für Kernnadel nach einem der Ansprüche 8 oder 9, weiter umfassend eine Steuerwelle (360, 560), wobei die Steuerwelle (360, 560) ein Betätigungselement (372, 572) einschließt, wobei das Betätigungselement (372, 572) dazu ausgebildet ist, mit dem Nockenelement (230, 430) des Rastmechanismus (220, 420) in Eingriff zu treten, um den Rastmechanismus (220, 420) relativ zu dem Axialpositionierelement (222, 422) zu drehen.

11. Biopsievorrichtung für Kernnadel nach einem der Ansprüche 1 bis 9, weiter umfassend eine Steuerwelle (360, 560), wobei die Steuerwelle (360, 560) dazu ausgebildet ist, sich relativ zu dem Rastmechanismus (220, 420) zu drehen, um mit dem Rastmechanismus (220, 420) in Eingriff zu treten und einen Teil der Schneidvorrichtungsantriebsanordnung (310, 510) selektiv von der Durchstechelementantriebsanordnung (210, 410) zu entkoppeln.

12. Biopsievorrichtung für Kernnadel nach einem der Ansprüche 1 bis 11, wobei die Gewindespindel dazu ausgebildet ist, eine axiale Verschiebung des Rastmechanismus (220, 420) anzutreiben.

## Revendications

1. Dispositif de biopsie à aiguille à noyau, comprenant :
(a) un ensemble à aiguille (20), l'ensemble à aiguille (20) incluant un perforateur (22) et un élément de coupe creux (40), le perforateur (22) incluant une pointe distale tranchante (24) et une encoche (26) à proximité de la pointe distale (24), le perforateur (22) étant disposé de manière coulissante à l'intérieur de l'élément de coupe (40) de manière à sectionner un échantillon de tissu dans l'encoche du perforateur (22) ;
(b) un ensemble (310, 510) d'entraînement de l'élément de coupe, configuré de manière à déplacer l'élément de coupe (40) ;
(c) un ensemble (210, 410) d'entraînement du perforateur, configuré de manière à déplacer le perforateur (22), l'ensemble (310, 410) d'entraînement du perforateur incluant une vis mère, la vis mère étant configurée de manière à déplacer à la fois une portion de l'ensemble (310, 510) d'entraînement de l'élément de coupe et une portion de l'ensemble (210, 410) d'entraînement du perforateur ; et
(d) un mécanisme de verrouillage (220, 420) configuré de manière à accoupler sélectivement une portion de l'ensemble (310, 510) d'entraînement de l'élément de coupe et une portion de l'ensemble (210, 410) d'entraînement du perforateur,
la vis mère incluant une vis mère (240, 440) du perforateur, incluant un premier filetage (242, 442) et une vis mère (250, 450) de l'élément de coupe incluant un deuxième filetage (252, 452), la vis mère (240, 440) du perforateur étant configurée de manière à entraîner la translation du perforateur (22), la vis mère (250, 450) de l'élément de coupe étant configurée de manière à entraîner la translation de l'élément de coupe (40),
**caractérisé en ce que** le deuxième filetage (252, 452) est un filetage de sens opposé au premier filetage (242, 442) de telle sorte que la rotation de la vis mère dans une seule direction entraîne la translation du perforateur (22) et de l'élément de coupe (40) dans des directions opposées.

2. Dispositif de biopsie à aiguille à noyau selon la revendication 1, le premier filetage (242, 442) présentant un pas grossier par rapport au deuxième filetage (252, 452).

3. Dispositif de biopsie à aiguille à noyau selon la revendication 2, la vis mère (240) du perforateur et la vis mère (250) de l'élément de coupe étant toutes deux externes par rapport à une structure de la vis mère.

4. Dispositif de biopsie à aiguille à noyau selon l'une quelconque des revendications 2 à 3, la vis mère (440) du perforateur ou la vis mère (450) de l'élément de coupe étant interne par rapport à une structure de la vis mère.

5. Dispositif de biopsie à aiguille à noyau selon l'une quelconque des revendications 1 à 4, la vis mère étant orientée le long d'un axe décalé par rapport à un axe longitudinal défini par l'ensemble à aiguille (20).

6. Dispositif de biopsie à aiguille à noyau selon l'une quelconque des revendications 1 à 4, la vis mère étant orientée le long d'un axe longitudinal défini par l'ensemble à aiguille (20).

7. Dispositif de biopsie à aiguille à noyau selon l'une quelconque des revendications 1 à 6, l'ensemble (310, 510) d'entraînement de l'élément de coupe, l'ensemble (210, 410) d'entraînement du perforateur, et le mécanisme de verrouillage (220, 420) étant orientés selon un axe unique, le mécanisme de verrouillage (220, 420) étant disposé entre une portion de l'ensemble (310, 510) d'entraînement de l'élément de coupe et de l'ensemble (210, 410) d'entraînement du perforateur.

8. Dispositif de biopsie à aiguille à noyau selon l'une quelconque des revendications 1 à 7, le mécanisme de verrouillage (220, 420) incluant un organe (222, 422) de positionnement axial et un organe (230, 430) formant came, l'organe (230, 430) formant came étant configuré de manière à tourner par rapport à l'organe (222, 422) de positionnement axial de manière à désaccoupler sélectivement une portion de l'ensemble (310, 510) d'entraînement de l'élément de coupe par rapport à une portion de l'ensemble (210, 410) d'entraînement du perforateur.

9. Dispositif de biopsie à aiguille à noyau selon la revendication 8, l'organe (230, 430) formant came du mécanisme de verrouillage (220, 420) incluant un ou plusieurs éléments (236, 436) formant came, lesdits un ou plusieurs éléments (236, 436) formant came étant configurés de manière à manœuvrer une portion de l'ensemble (310, 510) d'entraînement de l'élément de coupe lors de la rotation de l'organe (230, 430) formant came par rapport à l'organe (222, 422) de positionnement axial.

10. Dispositif de biopsie à aiguille à noyau selon les revendications 8 ou 9, comprenant en outre un arbre de commande (360, 560), l'arbre de commande (360, 560) incluant un actionneur (372, 572), l'actionneur (372, 572) étant configuré de manière à venir en prise avec l'organe (230, 430) formant came du mécanisme de verrouillage (220, 420) afin de faire tourner le mécanisme de verrouillage (220, 420) par rapport à l'organe (222, 422) de positionnement axial.

11. Dispositif de biopsie à aiguille à noyau selon l'une quelconque des revendications 1 à 9, comprenant en outre un arbre de commande (360, 560), l'arbre de commande (360, 560) étant configuré de manière à tourner par rapport au mécanisme de verrouillage (220, 420) afin de venir en prise avec le mécanisme de verrouillage (220, 420) et de désaccoupler sélectivement une portion de l'ensemble (310, 510) d'entraînement de l'élément de coupe d'avec l'ensemble (210, 410) d'entraînement du perforateur.

12. Dispositif de biopsie à aiguille à noyau selon l'une quelconque des revendications 1 à 11, la vis mère étant configurée de manière à entraîner la translation axiale du mécanisme de verrouillage (220, 420).
